# EUROPEAN PATENT APPLICATION

(11) **EP 3 745 346 A1**
(43) Date of publication of application: **02.12.2020**
(21) Application number: 18901964.9
(22) Date of filing: 30.10.2018
(51) Int. Cl.: G06Q 50/10

(54) **INFORMATION PROCESSING DEVICE, INFORMATION PROCESSING METHOD, AND RECORDING MEDIUM**

(30) Priority: 23.01.2018 JP 2018008607
(71) Applicant: Sony Corporation, 108-0075 Tokyo (JP)
(72) Inventor: MIYAJIMA, Yasushi, Tokyo 105-0014 (JP); SHIONOZAKI, Atsushi, Tokyo 105-0014 (JP); IWANAMI, Hiroshi, Tokyo 105-0014 (JP)
(74) Representative: MFG Patentanwälte Meyer-Wildhagen Meggle-Freund Gerhard PartG mbB
(86) International application number: PCT/JP2018/040269
(87) International publication number: WO 2019/146200

(57) **Abstract**

[Problem to be Solved] To provide an information processing apparatus, an information processing method, and a recording medium that are able to automatically generate a behavior rule of a community and to promote voluntary behavior modification

[Solution] An information processing apparatus including a controller that acquires sensor data obtained by sensing a member belonging to a specific community, automatically generates, on a basis of the acquired sensor data, a behavior rule in the specific community, and performs control to prompt, on a basis of the behavior rule, the member to perform behavior modification.

## Description

### Technical Field

The present disclosure relates to an information processing apparatus, an information processing method, and a recording medium.

### Background Art

Recently, an agent has been provided that makes recommendation of contents and behaviors corresponding to a user's question, request, and context by using a dedicated terminal such as a smartphone, a tablet terminal, or a home agent. Such an agent has been designed to improve the user's short-term convenience and comfort at a present time. For example, an agent that answers a weather, sets an alarm clock, or manages a schedule when asking a question is closed in one short-term session (completed by a request and a response) in which a response to a question or an issue is direct and short-term.

In contrast, there are the following existing techniques for promoting behavior modification for gradually approaching a solution to an issue from a long-term perspective.

For example, PTL 1 below discloses a behavior support system including a means for determining which of behavior modification stages a subject corresponds to from targets and behavior data of the subject in the fields of healthcare, education, rehabilitation, autism treatment, and the like, and a means for selecting a method of interventions for performing behavior modification on the subject on the basis of the determination.

Further, PTL 2 below discloses a support device for automatically determining behavior modification stages by an evaluation unit having evaluation conditions of evaluation rules, which are automatically generated using data for learning. Specifically, it is possible to determine a behavior modification stage from a conversation between a metabolic syndrome guidance leader and a subject.

### Citation List

### Patent Literature

PTL 1: Japanese Unexamined Patent Application Publication No. 2016-85703
PTL 2: Japanese Unexamined Patent Application Publication No. 2010-102643

### Summary of the Invention

### Problems to be Solved by the Invention

However, in each of the above-mentioned existing techniques, a specific issue has been decided in advance, and the issue itself has not been determined. Further, the stages of the behavior modification have also been decided in advance and it has only been possible to determine a specific event on a rule-based basis.

Further, regarding specific communities such families, the smaller the group, the higher the possibility that the behavior rules differ among the communities, but generation of a behavior rule for each specific community has not been carried out.

Accordingly, the present disclosure proposes an information processing apparatus, an information processing method, and a recording medium that are able to automatically generate a behavior rule of a community and to promote voluntary behavior modification.

### Means for Solving the Problems

According to the present disclosure, there is proposed an information processing apparatus including a controller that acquires sensor data obtained by sensing a member belonging to a specific community, automatically generates, on a basis of the acquired sensor data, a behavior rule in the specific community, and performs control to prompt, on a basis of the behavior rule, the member to perform behavior modification.

According to the present disclosure, there is provided an information processing apparatus including a controller that encourages a member belonging to a specific community to perform behavior modification, depending on a behavior rule in the specific community, the behavior rule being automatically generated in advance on a basis of sensor data obtained by sensing the member belonging to the specific community, in accordance with the sensor data obtained by sensing the member belonging to the specific community.

According to the present disclosure, there is provided an information processing method performed by a processor, the method including acquiring sensor data obtained by sensing a member belonging to a specific community, automatically generating, on a basis of the acquired sensor data, a behavior rule in the specific community, and performing control to prompt, on a basis of the behavior rule, the member to perform behavior modification.

According to the present disclosure, there is provided a recording medium having a program recorded therein, the program causing a computer to function as a controller that acquires sensor data obtained by sensing a member belonging to a specific community, automatically generates, on a basis of the acquired sensor data, a behavior rule in the specific community, and performs control to prompt, on a basis of the behavior rule, the member to perform behavior modification.

### Effects of the Invention

As described above, according to the present disclosure, it is possible to automatically generate a behavior rule of a community and to promote voluntary behavior modification.

It is to be noted that the effects described above are not necessarily limitative. With or in the place of the above effects, there may be achieved any one of the effects described in this specification or other effects that may be grasped from this specification.

### Brief Description of Drawings

[FIG. 1] FIG. 1 is a diagram explaining an outline of an information processing system according to an embodiment of the present disclosure.
[FIG. 2] FIG. 2 is a block diagram illustrating an example of a configuration of the information processing system according to an embodiment of the present disclosure.
[FIG. 3] FIG. 3 is a flowchart of an operation process of the information processing system according to an embodiment of the present disclosure.
[FIG. 4] FIG. 4 is a diagram explaining an outline of a master system of a first working example according to the present embodiment.
[FIG. 5] FIG. 5 is a block diagram illustrating a configuration example of the master system of the first working example according to the present embodiment.
[FIG. 6] FIG. 6 is a diagram illustrating examples of issue indices of the first working example according to the present embodiment.
[FIG. 7] FIG. 7 is a diagram explaining a causality analysis of the first working example according to the present embodiment.
[FIG. 8] FIG. 8 is a diagram explaining a causal path search based on a causality analysis result of the first working example according to the present embodiment.
[FIG. 9] FIG. 9 is a table of a probability distribution between a breakfast-start time and a gathering time period (hour(s)/week) of the first working example according to the present embodiment.
[FIG. 10] FIG. 10 is a probability distribution between a wake-up time and the breakfast-start time of the first working example according to the present embodiment.
[FIG. 11] FIG. 11 is a diagram explaining a matrix operation for determining a probability distribution between the wake-up time and the gathering time period of the first working example according to the present embodiment.
[FIG. 12] FIG. 12 is a diagram illustrating a table of the probability distribution between the wake-up time and the gathering time period obtained as a result of the matrix operation illustrated in FIG. 11.
[FIG. 13] FIG. 13 is a flowchart of an overall flow of an operation process of the first working example according to the present embodiment.
[FIG. 14] FIG. 14 is a flowchart of an issue estimation process of the first working example according to the present embodiment.
[FIG. 15] FIG. 15 is a flowchart of an intervention reservation process of the first working example according to the present embodiment.
[FIG. 16] FIG. 16 is a flowchart of an intervention process of the first working example according to the present embodiment.
[FIG. 17] FIG. 17 is a diagram illustrating some examples of causal paths of a values gap of the first working example according to the present embodiment.
[FIG. 18] FIG. 18 is a block diagram illustrating an example of a configuration of a master system of a second working example according to the present embodiment.
[FIG. 19] FIG. 19 is a basic flowchart of an operation process of the second working example according to the present embodiment.
[FIG. 20] FIG. 20 is a flowchart of a behavior modification process related to meal discipline of the second working example according to the present embodiment.
[FIG. 21] FIG. 21 is a flowchart of a behavior modification process related to putting away of plates of the second working example according to the present embodiment.
[FIG. 22] FIG. 22 is a flowchart of a behavior modification process related to clearing up of a desk of the second working example according to the present embodiment.
[FIG. 23] FIG. 23 is a flowchart of a behavior modification process related to tidying up of a room of the second working example according to the present embodiment.
[FIG. 24] FIG. 24 is a diagram explaining an example of information presentation that promotes the behavior modification of the example illustrated in FIG. 23.
[FIG. 25] FIG. 25 is a flowchart of a behavior modification process related to a baby cry of the second working example according to the present embodiment.
[FIG. 26] FIG. 26 is a flowchart of a behavior modification process related to a toy of the second working example according to the present embodiment.
[FIG. 27] FIG. 27 is a flowchart of a behavior modification process related to a general sense of values of the second working example according to the present embodiment.
[FIG. 28] FIG. 28 is a table of a calculation example of each candidate for the general sense of values of the second working example according to the present embodiment.
[FIG. 29] FIG. 29 is a block diagram illustrating an example of a configuration of a master system of a third working example according to the present embodiment.
[FIG. 30] FIG. 30 is a graph of an example of a food record of the third working example according to the present embodiment.
[FIG. 31] FIG. 31 is a diagram explaining a deviation in a life rhythm of the third working example according to the present embodiment.
[FIG. 32] FIG. 32 is a flowchart of an operation process of generating a rhythm of an evening meal time of the third working example according to the present embodiment.
[FIG. 33] FIG. 33 is a diagram illustrating an example of a formula for calculating an accumulated average time for each day of the week of the third working example according to the present embodiment.
[FIG. 34] FIG. 34 is a flowchart for generating advice on the basis of the life rhythm of the third working example according to the present embodiment.
[FIG. 35] FIG. 35 is a flowchart for promoting adjustment (behavior modification) of the life rhythm in accordance with overlapping of an event according to a modification example of the third working example of the present embodiment.
[FIG. 36] FIG. 36 is a block diagram illustrating an example of a hardware configuration of an information processing apparatus according to the present embodiment.

### Modes for Carrying Out the Invention

The following describes a preferred embodiment of the present disclosure in detail with reference to the accompanying drawings. It is to be noted that, in this description and the accompanying drawings, components that have substantially the same functional configuration are indicated by the same reference signs, and thus redundant description thereof is omitted.

It is to be noted that description is given in the following order.
1. Outline of Information Processing System According to Embodiment of Present Disclosure
2. First Working Example (Estimation of Issue and Behavior Modification)
   2-1. Configuration Example
   2-2. Operation Process
   2-3. Supplement
3. Second Working Example (Generation of Standard of Value and Behavior Modification)
   3-1. Configuration Example
   3-2. Operation Process
4. Third Working Example (Adjustment of Life Rhythm)
   4-1. Configuration Example
   4-2. Operation Process
   4-3. Modification Example
5. Hardware Configuration Example
6. Conclusion

### [1. Outline of Information Processing System According to Embodiment of Present Disclosure]

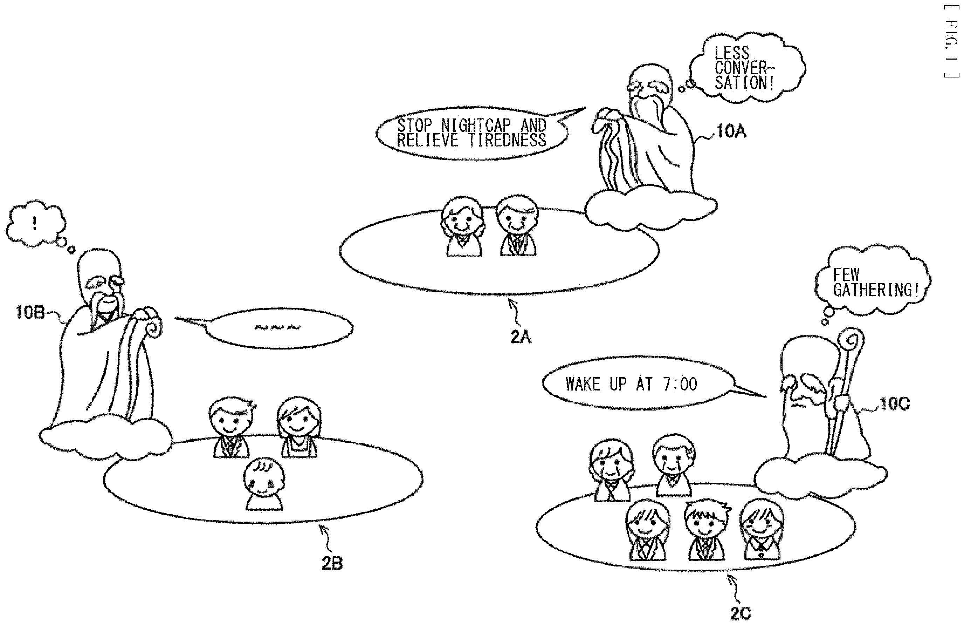

FIG. 1 is a diagram for explaining an outline of an information processing system according to an embodiment of the present disclosure. As illustrated in FIG. 1, in the information processing system according to the present embodiment, master systems 10A to 10C are present each of which promotes behavior modification through a virtual agent (playing a role of a master of a specific community, hereinafter referred to as "master" in this specification) in accordance with a predetermined behavior rule for corresponding one of communities 2A to 2C such as families. In FIG. 1, the master systems 10A to 10C are each illustrated as a person. The master systems 10A to 10C may each automatically generate a behavior rule on the basis of a behavior record of each user in a specific community, indirectly promote behavior modification on the basis of the behavior rule, and thus perform issue solving of the community, and the like. As for the user, while behaving in accordance with words of the master, it is possible, without being aware of the behavior rule (an issue or a standard of value), to solve the issue in the community and to adjust the standard of value before anyone knows, which allows a situation of the community to be improved.

### [Background]

As described above, in each of the existing master systems, the specific issue has been decided in advance, and the issue itself has not been determined. In addition, although the existing master systems are each closed in one short-term session which is completed by a request and a response, there are many issues in which a plurality of factors are complicatedly intertwined in real life, and it is not possible that such issues are solved directly or in a short term.

Contents and solving methods of the issues are not the same, and for example, it is considered that in cases of household problems, degrees of seriousness and solving methods of an issue may differ depending on behavior rules and environments of the process. For this reason, it is important to analyze a relationship among multiple factors to explore a long-term, not a short-term solution, and to explore where to intervene. Taking behavior rules as an example, in specific communities such families, the smaller the group, the higher the possibility that the behavior rules differ among the communities, and the more likely there is "ethics" unique to each of the communities. Thus, it is not possible to restrict a behavior rule to one general-purpose object or to use collected entire big data as a standard; therefore, it becomes important to collect data focusing on a specific community such as a family, or the like, and to clarify the behavior rule in the specific community.

Accordingly, as illustrated in FIG. 1, the present embodiment provides a master system 10 that is able to automatically generate a behavior rule for each specific community and to promote voluntary behavior modification.

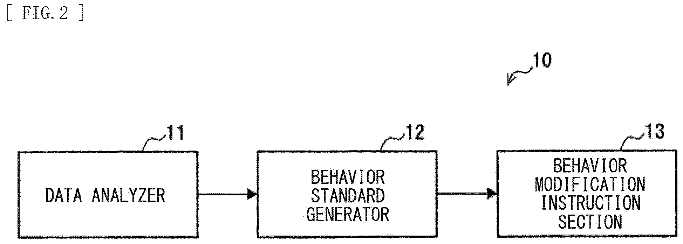

FIG. 2 is a block diagram illustrating an example of a configuration of the information processing system (master system 10) according to an embodiment of the present disclosure. As illustrated in FIG. 2, the master system 10 according to the present embodiment includes a data analyzer 11, a behavior rule generator 12, and a behavior modification instruction section 13. The master system 10 may be a server on a network, or may be a client device including: a dedicated terminal such as a home agent; a smartphone; a tablet; and the like.

The data analyzer 11 analyzes sensing data obtained by sensing a behavior of a user belonging to a specific community such as a family.

The behavior rule generator 12 generates a behavior rule of the specific community on the basis of an analysis result obtained by the data analyzer 11. Here, the "behavior rule" includes a means for solving an issue that the specific community has (for example, estimating an issue that a gathering time period is small and automatically generating a behavior rule that causes a gathering time period to be increased), or generation (estimation) of a standard of value in the specific community.

The behavior modification instruction section 13 controls a notification or the like that prompts the user of the specific community to perform behavior modification in accordance with the behavior rule generated by the behavior rule generator 12.

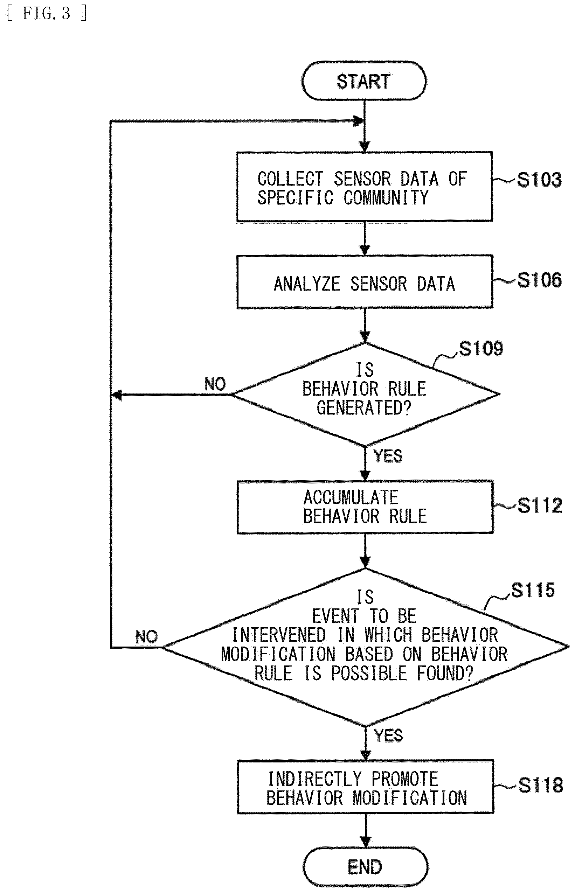

An operation process of the master system 10 having such a configuration is illustrated in FIG. 3. As illustrated in FIG. 3, first, the master system 10 collects sensor data of the specific community (step S103) and analyzes the sensor data by the data analyzer 11 (step S106).

Next, the behavior rule generator 12 generates a behavior rule of the specific community on the basis of the analysis result obtained by the data analyzer 11 (step S109), and in a case where the behavior rule generator 12 has been able to generate the behavior rule (step S109/Yes), accumulates information of the behavior rule data (step S112).

Subsequently, the behavior modification instruction section 13 determines an event to be intervened in which behavior modification based on the behavior rule is possible (step S115/Yes). For example, determination of event (a wake-up time, an exercise frequency, or the like, or a life rhythm) in which the behavior modification is possible for achieving the behavior rule or a situation deviating from the standard of value is determined as the event to be intervened.

Thereafter, in a case where the event to be intervened in which the behavior modification is possible is found (step S115/Yes), the behavior modification instruction section 13 indirectly prompts the user in the specific community to perform behavior modification (step S118). Specifically, the behavior modification instruction section 13 indirectly promotes a change in the behavior, an adjustment in the life rhythm, or a behavior that solves a deviation from the standard of value. By automatically generating the behavior rule and indirectly promoting the behavior modification, each user in the specific community is able, while behaving in accordance with the master, without being aware of the behavior rule (an issue or a standard of value), to solve the issue in the specific community and to take a behavior according to the standard of value before anyone knows, which allows a situation of the specific community to be improved.

The outline of the information processing system according to an embodiment of the present disclosure has been described above. It is to be noted that the configuration of the present embodiment is not limited to the example illustrated in FIG. 2. For example, in a case where the a behavior rule in a specific community automatically generated in advance on the basis of sensor data obtained by sensing a member belonging to the specific community has been already possessed, an information processing apparatus may be used that performs control to encourage the member to perform behavior modification (the behavior modification instruction section 13) in accordance with the sensor data obtained by sensing the member belonging to the specific community (the data analyzer 11). Next, the information processing system according to the present embodiment will be described specifically with reference to first to third working examples. A first working example describes that "an analysis of a means for solving an estimated issue" is performed for generating a behavior rule, and behavior modification is indirectly promoted to solve an issue. Further, a second working example describes that "generation of a standard of value" is performed for generating a behavior rule, and behavior modification is indirectly promoted in a case of deviating from the standard of value. Still further, a third working example describes that a life rhythm is adjusted, as the behavior modification for solving the issue estimated in the first working example.

### [2. First Working Example (Estimation of Issue and Behavior Modification)]

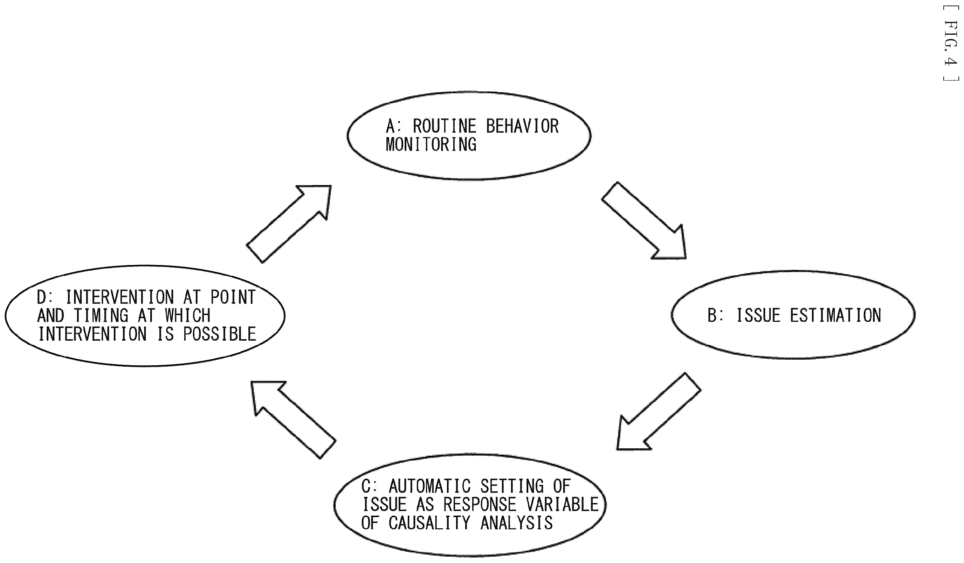

First, referring to FIGs. 4 to 17, a master system 10-1 according to the first working example will be described. In the present embodiment, routine data collection and routine analyses are performed (Casual Analysis) on a small community such as on a family basis, to identify an issue occurring in the family and to perform an intervention that promotes behavior modification to solve the issue from a long-term perspective. That is, the issue of the family is estimated on the basis of the routinely collected family data, an response variable is automatically generated as a behavior rule for solving the issue (e.g., "(increase) a gathering time period"), a relationship graph of factor variables having the response variable as a start point is created, an intervention point (e.g., "late-night amount of alcohol drinking" or "exercise strength"; a factor variable), which is a point in which the behavior modification is promoted to cause the response variable to have a desired value, is detected and intervened, and the issue of the family is lead to a solution over a long-term span (e.g., prompting members to perform behavior modification so that the factor variable associated with the response variable approaches a desired value). For an analysis algorithm, CALC (registered trademark), which is a causality analysis algorithm provided by Sony Computer Science Laboratories, Inc., may be used; thus, it is possible to analyze a complex causal relationship between many variables.

FIG. 4 is a diagram explaining an outline of the first working example. The first working example is performed roughly by a flow illustrated in FIG. 4. That is, A: routine behavior monitoring is performed, B: issue estimation is performed, C: an issue is automatically set as a response variable of a causality analysis, D: intervention is performed at a point and a timing at which an intervention is possible (behavior modification is promoted). By routinely repeating the processes of A to D, the behavior modification is performed, and the issue in the specific community is gradually solved.

### [A: Routine Behavior Monitoring]

In the present embodiment, with an increase in the number of pieces of information that can be sensed, the larger range of data can be used for analysis. The data to be analyzed is not limited to specific types of data. For example, in the existing techniques or the like, data to be used for the analysis has been determined in advance by restricting the application; however, this is not necessary in the present embodiment, and it is possible to increase the types of data that can be acquired as occasion arises (registered in a database as occasion arises).

### [B: Issue Estimation]

In the present embodiment, an issue may be estimated using an index list (for example, an index list related to family closeness) that describes a relationship between an index that can be an issue related to the family, which is an example of a specific community, and an index of a sensor or a behavior history necessary for calculating the index. Specifically, values of the respective sensor/behavior history indices are checked to determine whether the index that can be an issue (e.g., "gathering time period of the family") is below (or above) a threshold. This process is performed for the same number of times as the number of items in the list, and it becomes possible to list the issues held by the family.

### [C: Automatic Setting of Issue as Response variable of Causality Analysis]

The causality analysis is performed using the detected issue as a response variable and other sensor/behavior history information as an explanatory variable. In this case, not only the index related to the issue in the index list but also other indices may be inputted as the explanatory variables. In a case where there is a plurality of issues, the issues are individually analyzed for a plurality of times, each as a response variable.

### [D: Master Intervention at Point and Timing at Which Intervention Is Possible]

A result obtained by the analysis has a graphical structure in which a factor directly related to a response variable is coupled to the response variable and another factor is further coupled to the factor. By tracing the graph from the response variable as a start point, it becomes possible to examine the causal relationship retroactively in a direction from the result to the cause. At this time, each factor has a flag that indicates whether or not each factor is an intervention-available factor (e.g., wake-up time), and if the intervention is possible, the master intervenes on the factor to promote the behavior modification to improve the result. As a method of promoting the behavior modification, in addition to a method of directly issuing an instruction to the user, it is also possible to perform indirect interventions such as playing a relaxing music and setting the wake-up time to an optimal time.

### [2-1. Configuration Example]

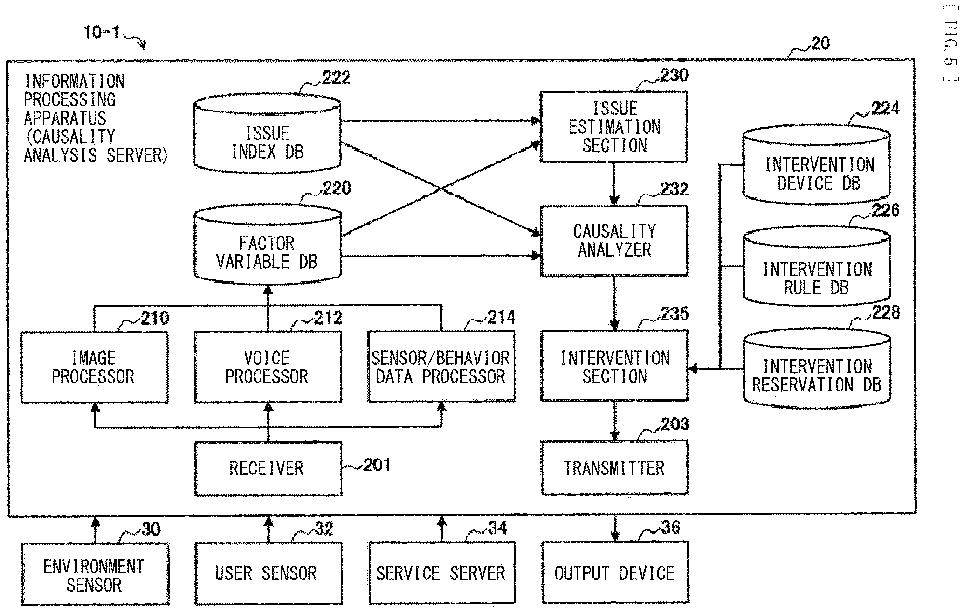

FIG. 5 is a block diagram illustrating a configuration example of the master system 10-1 of the first working example. As illustrated in FIG. 5, the master system 10-1 includes an information processing apparatus 20, an environment sensor 30, a user sensor 32, a service server 34 and an output device 36.

### [Sensor Group]

The environment sensor 30, the user sensor 32, and the service server 34 are examples from which information about a user (member) belonging to a specific community is acquired, and the present embodiment is not limited thereto and is not limited to a configuration that includes all of those.

The environment sensor 30 includes, for example, a camera installed in a room, a microphone (hereinafter, referred to as a microphone), a distance sensor, an illuminance sensor, various sensors provided on the environment side such as a pressure/vibration sensor installed on a table or a chair, a bed, and the like. The environment sensor 30 performs detection on a per-community basis, and, for example, determines an amount of smile with a fixed camera in a living room, which makes it possible to acquire the amount of smile under a single condition in the home.

The user sensor 32 includes various sensors such as an acceleration sensor, a gyro sensor, a geomagnetic sensor, a position sensor, a biological sensor of a heart rate, a body temperature, or the like, a camera, a microphone, and the like provided in a smartphone, a mobile phone terminal, a tablet terminal, a wearable device (HMD, smart glasses, a smart band, or the like) or the like.

Assumed as the service server 34 are an SNS server, a positional information acquisition server, and an e-commerce server (e.g., an e-commerce site) that are used by the user belonging to the specific community, and the service server 34 may acquire, from a network, information related to the user (information and the like related to user's behavior such as a move history and a shopping history) other than information acquired by the sensor.

### [Information Processing Apparatus 20]

The information processing apparatus 20 (causality analysis server) includes a receiver 201, a transmitter 203, an image processor 210, a voice processor 212, a sensor/behavior data processor 214, a factor variable DB (database) 220, an intervention device DB 224, an intervention rule DB 226, an intervention reservation DB 228, an issue index DB 222, an issue estimation section 230, a causality analyzer 232, and an intervention section 235. The image processor 210, the voice processor 212, the sensor/behavior data processor 214, the issue estimation section 230, the causality analyzer 232, and the intervention section 235 may be controlled by a controller provided to the information processing apparatus 20. The controller functions as an arithmetic processing unit and a control unit, and controls overall operations in the information processing apparatus 20 in accordance with various programs. The controller is achieved by, for example, an electronic circuit such as CPU (Central Processing Unit) or a microprocessor. Further, the controller may include a ROM (Read Only Memory) that stores programs, operation parameters, and the like to be used, and a RAM (Random Access Memory) that temporarily stores parameters and the like that vary as appropriate.

The information processing apparatus 20 may be a cloud server on the network, may be an intermediate server or an edge server, may be a dedicated terminal located in a home such as a home agent, or may be an information processing terminal such as a PC or a smartphone.

### -Receiver 201 and Transmitter 203

The receiver 201 acquires sensor information and behavior data of each user belonging to the specific community from the environment sensor 30, the user sensor 32, and the service server 34. The transmitter 203 transmits, to the output device 36, a control signal that issues an instruction of output control for indirectly promoting behavior modification in accordance with a process performed by the intervention section 235.

The receiver 201 and the transmitter 203 are configured by a communication section (not illustrated) provided to the information processing apparatus 20. The communication section is coupled via wire or radio to external devices such as the environment sensor 30, the user sensor 32, the service server 34, and the output device 36, and transmits and receives data. The communication section communicates with the external devices by, for example, a wired/wireless LAN (Local Area Network), or Wi-Fi (registered trademark), Bluetooth (registered trademark), a mobile communication network (LTE (Long Term Evolution), 3G (third-generation mobile communication system)), or the like. -Data Processor

Various pieces of sensor information and behavior data of the user belonging to the specific community are appropriately processed by the image processor 210, the voice processor 212, and the sensor/behavior data processor 214. Specifically, the image processor 210 performs person recognition, expression recognition, object recognition, and the like on the basis of an image captured by a camera. Further, the voice processor 212 performs conversation recognition, speaker recognition, positive/negative recognition of conversation, emotion recognition, etc. on the basis of a voice collected by the microphone. In addition, the sensor/behavior data processor 214 performs a process such as converting raw data into meaningful labels by performing a process instead of recording the raw data as it is depending on the sensor (for example, converting the raw data into a seating time period on the basis of a chair vibration sensor). Moreover, the sensor/behavior data processor 214 extracts, from SNS information and positional information (e.g., GPS), user's behavior contexts (e.g., a meal at a restaurant with family) indicating where and doing what. Still further, the sensor/behavior data processor 214 is also able to extract positive/negative of emotion from sentences posted to the SNS, and extract information such as who the user is with and who the user is in sympathy with from interaction information between users.

The data thus processed is stored in the factor variable DB 220 as variables for issue estimation and causality analysis. The variables stored in the factor variable DB 220 are each referred to as "factor variable" hereafter. Examples of the factor variable may include types indicated in the following Table 1; however, the types of the factor variable according to the present embodiment are not limited thereto, and any index that is obtainable may be used.

**[Table 1]**

| Original data | Examples of factor variable |
|---|---|
| Image | Person ID, expression, posture, behavior, clothes, object ID, degree of messiness of room, brightness of room, etc., |
| | and percentage variables (smile percentage and pajama percentage), average variables during certain period (average of brightness of room and average of degree of messiness of room), etc., based on the above factor variables |
| Voice | Person ID, emotion, positive/negative utterance, favorite phrase, frequent word, volume of voice, anxiety about voice recognition / preference / things he/she wants to do / places where he/she wants to go / things he/she wants / etc., |
| | and percentage variables (negative utterance percentage and favorite phrase percentage), average variables during certain period (average of volume of voice), etc., based on the above factor variables |
| Other sensors | Seating time period on chair based on vibration sensor on chair or table, temperature, humidity, body temperature, heart rate, sleep length, sleep quality (REM sleep, non-REM sleep, roll-over), exercise quantity, number of steps, alcohol drinking, etc., |
| | and percentage variables and average variables thereof |
| SNS/GPS, etc. | Behavior, behavior area, positive/negative utterance, emotion, amount of interaction with friend, etc., |
| | and percentage variables and average variables thereof |

### -Issue Estimation Section 230

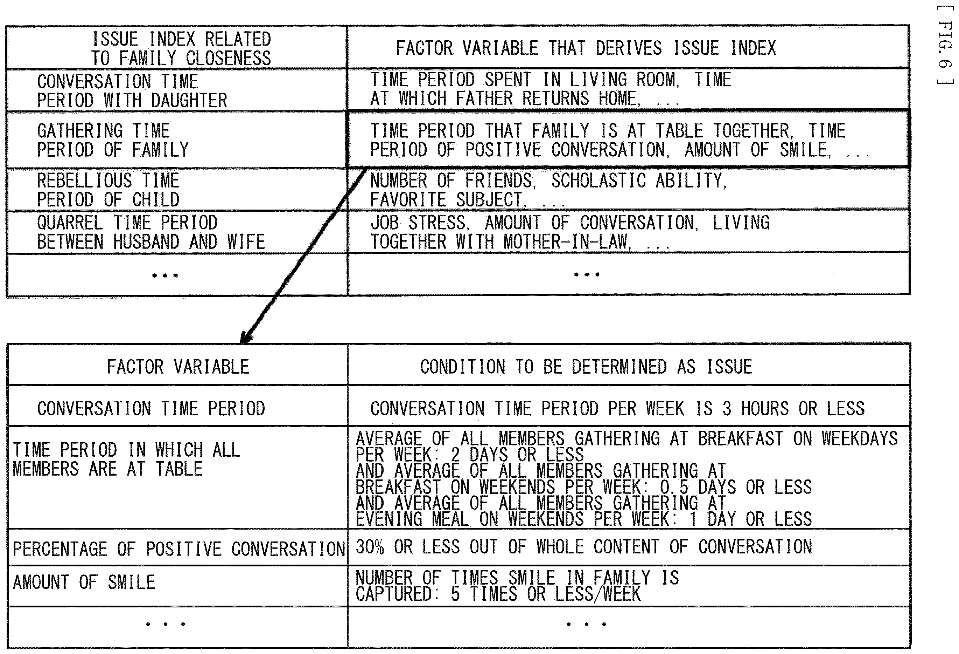

The issue estimation section 230 examines values of factor variables associated with respective issue indices registered in the issue index DB 222, and determines whether an issue has occurred (estimates an issue). FIG. 6 illustrates examples of issue indices according to the present embodiment. As illustrated in FIG. 6, for example, as issue indices related to family closeness, "conversation time period with daughter", "gathering time period of family", "rebellious time period of child", and "quarrel time period between husband and wife" are set in advance. Examples of factor variables of the respective issue indices include items as indicated in FIG. 6. Further, the issue index DB 222 is also associated with a condition for determining that there is an issue on the basis of the factor variable. For example, an issue of "gathering time period of family" is determined on the basis of a time period that the family is at the table together, a time period of positive conversation, an amount of smile, and the like, and more specifically, on the basis of the following conditions: "conversation time period per week is 3 hours or less", "all members gathering at breakfast on weekdays per week is 2 days or less", "percentage of positive conversation out of whole content of conversation is 30% or less", and the like.

The issue estimation section 230 may determine that there is an issue in a case where all the conditions presented in the issue index are satisfied, or may determine that there is an issue in a case where any one of the conditions is satisfied. In addition, it is permissible to set in advance whether to consider that there is an issue in the case where all the conditions are satisfied or to consider that there is an issue in the case where any one of the conditions is satisfied. It is also permissible to set a flag to set complex conditions for each issue. The factor variables used here are written in advance on the basis of a rule linked by a person with respect to the respective issue indices.

### -Causality Analyzer 232

The causality analyzer 232 performs causality analysis of an issue in a case where the issue estimation section 230 estimates the issue (determines that the issue has occurred). In the past, estimation of a statistical causal relationship based on data of observation in multivariate random variables is roughly divided into: a method of obtaining, as a score, a result of estimation obtained by an information criterion, a penalized maximum likelihood method, or a Bayesian method, and maximizing the score; and a method of performing estimation by a statistical test of conditional independence between variables. Representing the resulting causal relationship between variables as a graphical model (non-cyclic model) is often performed because of the readability of the result. Causality analysis algorithms are not particularly limited, and for example, the above-mentioned CALC provided by Sony Computer Science Laboratories, Inc., may be used. CALC is a technique that has already been commercialized as an analytical technique for a causal relationship in large-scale data (https://www.isid.co.jp/news/release/2017/0530.html,https://www.isid.co.jp/solution/calc.html).

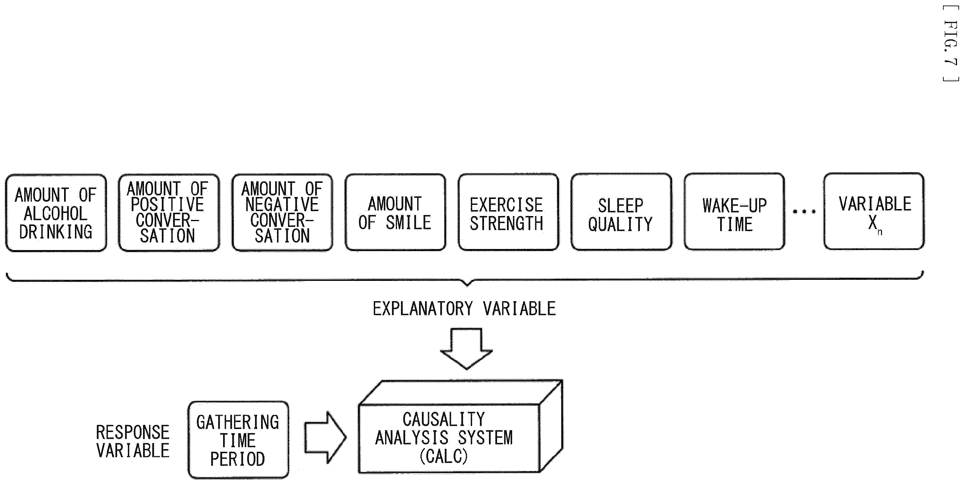

Specifically, the causality analyzer 232 sets an issue as a response variable and sets all or some of the rest of factor variables (basically it is better to include all, but the number of factor variables may be decreased due to a limitation on calculation time or memory, by selecting preferentially a factor variable whose number of pieces of data is larger or selecting preferentially a factor variable whose amount of data acquired recently is larger, for example) as explanatory variable(s), and the causality analysis is performed. FIG. 7 is a diagram explaining the causality analysis according to the present embodiment.

As illustrated in FIG. 7, in a case where an issue of "gathering time period" is estimated, this is set as a response variable. It is to be noted that in a case where it is not possible to directly set the variables stored in the factor variable DB 220 as the response variable, the response variable may be dynamically created. For example, since it is not possible to directly sense the "gathering time period", the response variable is generated by combining other variables. Specifically, variables such as a time period in which all family members are at the table at the same time, a time period in which positive conversations are being made, and a time period in which a percentage of a degree of smile is more than or equal to a certain value are combined, thereby deriving a total time period of joyful gathering, a quality of the gathering, and the like as the response variables. Rules of combining the variables may be stored in advance in the information processing apparatus 20 as a knowledge base, or may be automatically extracted.

The causality analyzer 232 outputs the analysis result to the intervention section 235.

### -Intervention Section 235

The intervention section 235 examines a causality analysis result, traces arrows backward from the factor variable that is directly connected to the response variable, and extracts a plurality of causal paths back until there are no more arrows to be traced. It is to be noted that the arrows are not necessarily present depending on the analysis method to be used, and in some cases, the simple straight lines may be used as a link. In such a case, the direction of the arrow is decided for convenience by utilizing characteristics of a causality analysis technique to be used. For example, a process may be performed by assuming that there is an arrow of convenience having a direction from a factor variable that is far (how many factor variables are between) from the response variable toward a factor variable that is closer to the response variable. In a case where a factor variable having the same distance from the response variable is coupled by a straight line, a direction of convenience is decided by taking into account the characteristics of the method used in the similar manner.

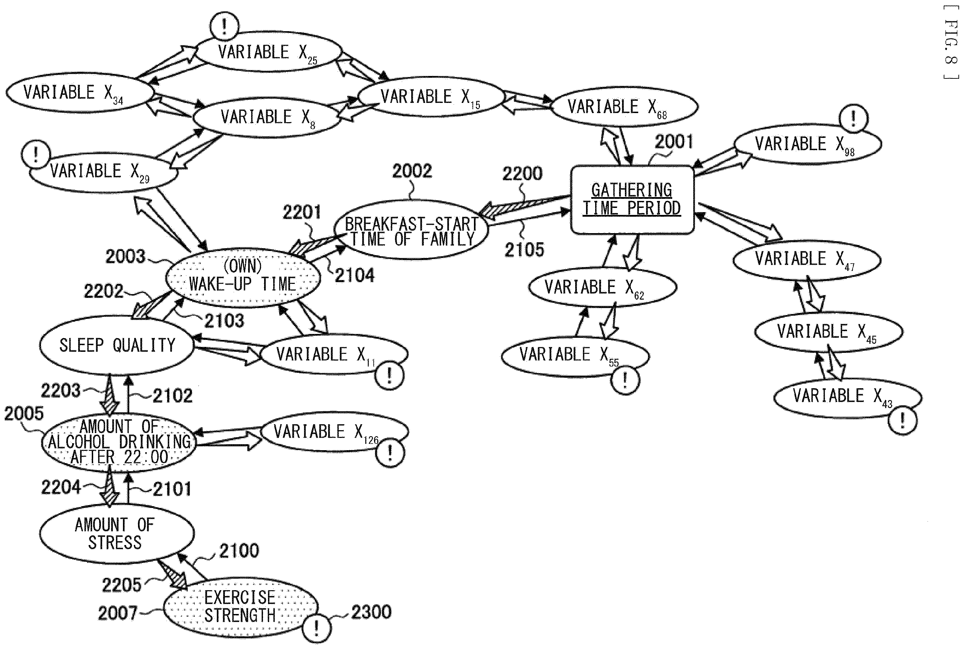

Here, FIG. 8 is a diagram explaining a causal path search based on the causality analysis result of the present embodiment. As illustrated in FIG. 8, the intervention section 235 traces a causal path (arrow 2105) coupled to "gathering time period" (response variable 2001) in the backward direction (arrow 2200) using "gathering time period" as a start point, for example, also traces a causal path (arrow 2104) coupled to the destination factor variable 2002 in the backward direction (arrow 2201), and such a causal path search is continued until there are no more arrows to be traced. That is, the arrows 2105 to 2100 of the causal paths illustrated in FIG. 8 are sequentially traced in the backward direction (arrows 2200 to 2205). At a time point when there are no more arrows to be traced in the backward direction (at a time point of reaching a factor variable 2007 to which a mark 2300 illustrated in FIG. 8 is imparted), the search for the path is terminated. In FIG. 8, it is possible to extract, as an example of the causal paths including factor variables, "exercise strength → amount of stress→ amount of alcohol drinking after 22:00→ sleep quality → wake-up time → breakfast-start time of family → gathering time period"; however, the intervention section 235 traces the arrows of such causal paths in the backward direction with "gathering time period" as the start point in the causal path search, and examines a relationship between a certain factor variable and the next factor variable at the upstream.

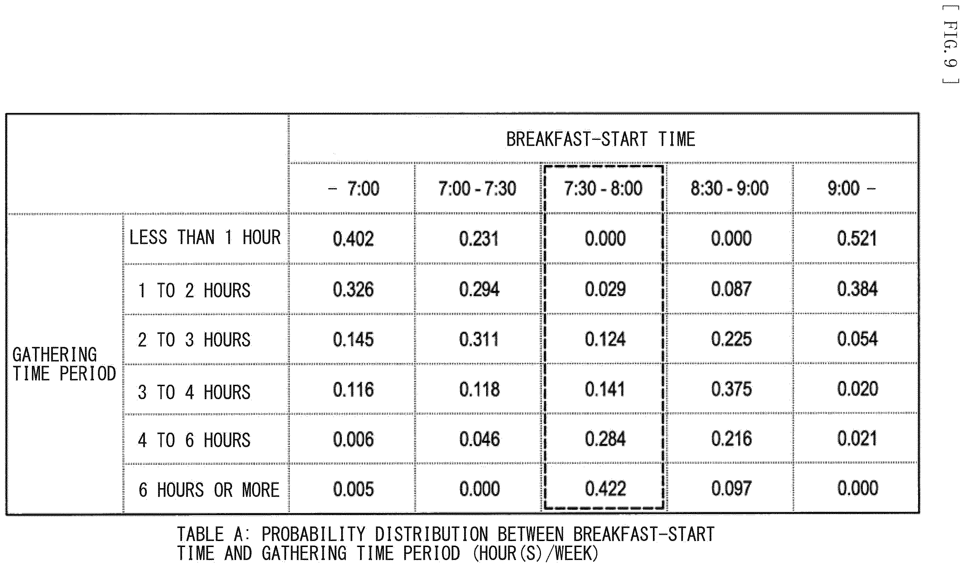

For example, the intervention section 235 sees "gathering time period", "breakfast-start time of family", "(own = father's) wake-up time" in probability distribution terms, and, in order to cause a value of the response variable to be within a target range, calculates the upstream factor variable that causes an expected value to have a highest value. The calculation of the expected value by a probability distribution calculation between such factor variables will be described referring to FIGs. 9 to 14. FIG. 9 is a table of a probability distribution between a breakfast-start time and gathering time period (hour(s)/week). The table in FIG. 9 indicates that the expected value of the gathering time period is highest when the breakfast is started between 7:30 and 8:00. Specifically, the gathering time period of the response variable (median values such as 0.5, 1.5, 2.5, 3.5, 5.0, and 6.0 are used as representative values because the gathering time period has a width) and the respective probabilities (0.000, 0.029, 0.124, 0.141, 0.284, and 0.442) are multiplied in order, and the sum thereof becomes the expected value of the gathering. In this example, 4.92 hours is the expected value. Determining the expected values by calculating the other time slots in the similar way, the gathering time period is the highest when the breakfast is started between 7:30 to 8:00 as a result.

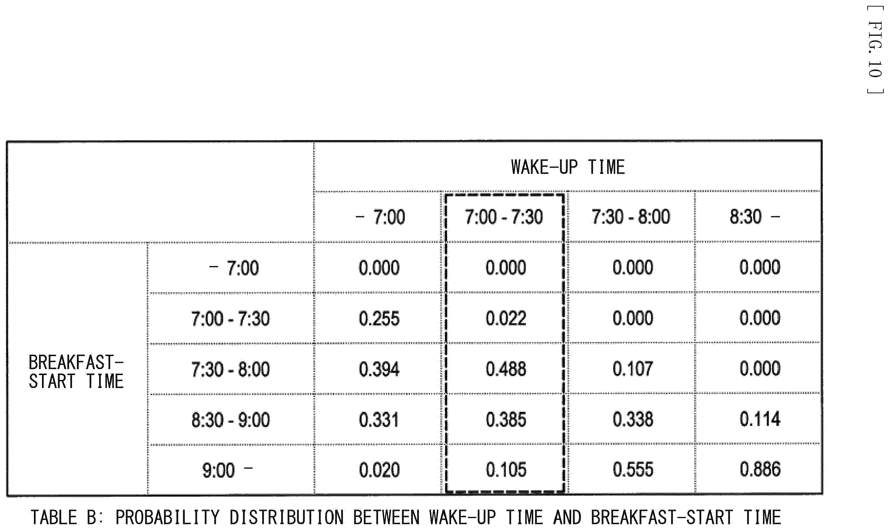

FIG. 10 is a probability distribution between a wake-up time and the breakfast-start time. According to the table in FIG. 10, it can be appreciated that the breakfast-start time is most likely to be between 7:30 and 8:00 when waking up between 7:00 to 7:30. It is to be noted that it is possible to determine the probability distribution between two coupled adjacent factor variables by performing cross tabulation of the original data.

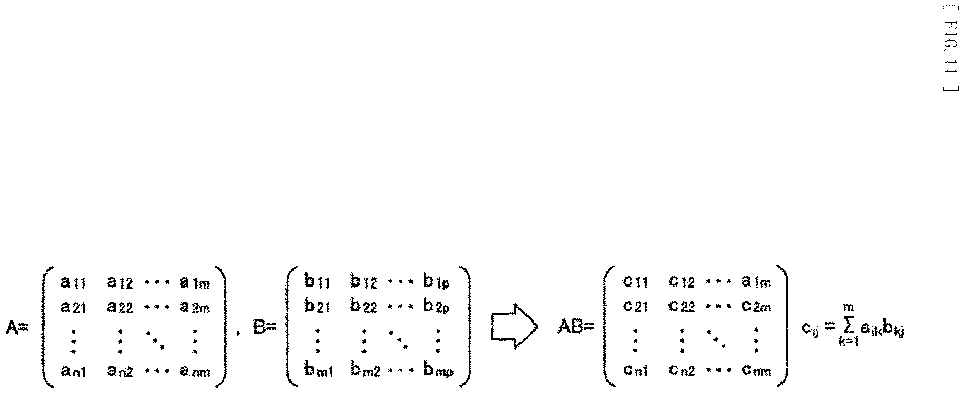

FIG. 11 is a diagram illustrating a matrix operation for determining a probability distribution between the wake-up time and the gathering time period. In a case where the table in FIG. 9 is represented by A and the table in FIG. 10 is represented by B, it is possible to determine the probability distribution between the wake-up time and the gathering time period by the matrix operation illustrated in FIG. 11.

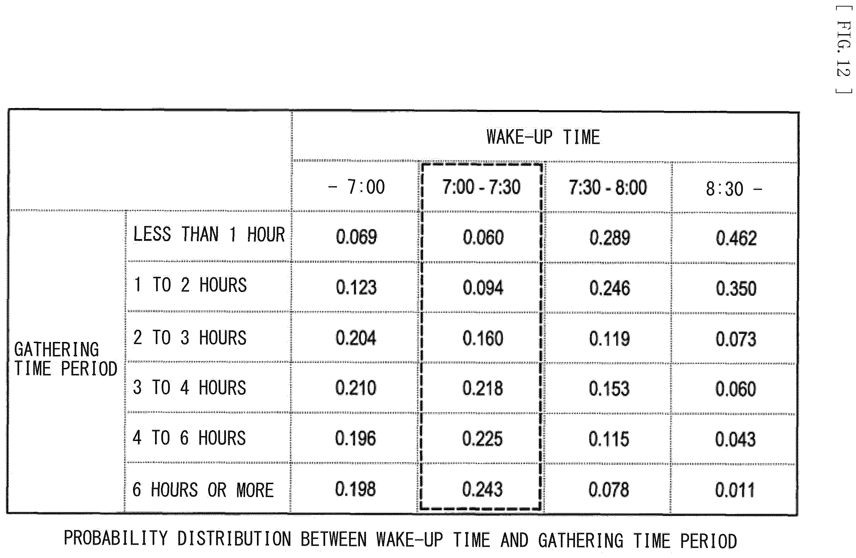

FIG. 12 is a diagram illustrating a table of the probability distribution between the wake-up time and the gathering time period obtained as a result of the matrix operation illustrated in FIG. 11. As illustrated in FIG. 12, waking up between 7:00 to 7:30, the gathering time period is more than 6 hours with a probability of 24.3%, and the gathering time period is more than 3 hours with a probability of approximately 70%. In contrast, waking up at 8:30 or later, the gathering time period is two hours or less at a rate of approximately 81%.

Thus, by repeating the multiplications of the conditional probability table toward the upstream, it is possible to find out which value the value of each factor variable of the causal path finally takes when the value of the response variable becomes the most targeted value.

It is to be noted that as an analysis method other than CALC, for example, it is also possible to use an analysis method called a Bayesian network that probabilistically expresses relationships among variables. In a case this method is applied, a variable (node) and an arrow (or line segment) coupling the variable (node) do not express a causal relationship, but the coupled variables are related to each other, and therefore, it is possible to apply the present embodiment as a convenient causality. In the present embodiment, even if the term "causality" is not used, it is possible to regard and apply the relationship between variables as a causality for convenience.

The intervention section 235 then searches the causal paths for a factor variable having an intervention-available flag, and acquires an intervention method for the factor variable from the intervention rule DB 226. When a factor variable is stored in the database, the factor variable is provided with a flag indicating an intervention availability. The flag may be provided by a person in advance, or the intervention-available may be provided in advance to raw sensor data, and if at least one piece of sensor data having the intervention-available flag is included when generating a factor variable, the factor variable may also be intervention-available. In the example illustrated in FIG. 8, for example, the factor variable 2003 (wake-up time), the factor variable 2005 (amount of alcohol drinking after 22:00), and the factor variable 2007 (exercise strength) are each provided with the intervention-available flag. For example, regarding the wake-up time, the intervention is available by an alarm setting of a wearable device; therefore, the wake-up time is registered in the database in a state of being provided with the intervention-available flag. As described above, in the present embodiment, it is possible to extract the intervention-available point from the factor variables on the way in which causal paths are traced backward from the response variable, and to perform indirect behavior modification (intervention). Here, some examples of the intervention rules registered in the intervention rule DB 226 are indicated in Table 2 below.

**[Table 2]**

| Factor variable | Intervention - available flag | Intervention method |
|---|---|---|
| Wake-up time | True | Automatically set alarm at appropriate date/time Target device capability: able to output any one of sound, vibration, or light at specified time |
| Sleep quality | False | N/A |
| Amount of stress | False | N/A |
| Exercise strength | True | Promote (suppress) sport or walking |
| | | Target device capability: cause application that promotes exercise to work, able to display coupon |
| Start time | False | N/A |

It is to be noted that actually the number of factor variables and the number of intervention methods are not infinite, and the intervention rules settle down to some degree of patterns, and hence may be shared by all agents in the cloud. However, parameters (wake-up time and target exercise strength) set at the time of the intervention may differ depending on individuals and families.

After acquiring the intervention method, the intervention section 235 searches intervention device DB 224 for a device having the target device capability. Devices that are available to the user or the family are registered in the intervention device DB 224. Here, some examples of the intervention devices are indicated in Table 3 below.

**[Table 3]**

| ID | Type | Owner | Device capability |
|---|---|---|---|
| 1 | TV | Shared | TV image reception, moving image playback, music playback, web browsing |
| 2 | Smartphone | Father | Application execution, music playback (time settable), moving image playback, vibration (time settable), web browsing, photo/moving image shooting, telephone call, push notification |
| 3 | Smartphone | Daughter | Application execution, music playback (time settable), moving image playback, vibration (time settable), web browsing, photo/moving image shooting, telephone call, push notification |
| 4 | Illumination | Shared | On/off, changing brightness (time settable), changing colors (time settable) |

When the intervention section 235 finds an appropriate device for intervention, the intervention section 235 registers a device ID, a triggering condition, an intervention command, and a parameter in the intervention reservation DB 228. Some examples of the intervention reservations are indicated in Table 4 below.

**[Table 4]**

| Reservation ID | Device ID | Condition | Command/parameter |
|---|---|---|---|
| 1 | 2 | 23:00, a day before a holiday | Start sound and vibration / 07:00, 29/9/2017 |
| 2 | 2 | 17:00 every Friday AND nothing in schedule | Display / coupon of commercial batting cage |

The intervention section 235 then transmits the commands and the parameter from the transmitter 203 to the specific device when the condition is met, based on the reserved data registered in the intervention reservation DB 228.

### [Output Device 36]

The output device 36 is a device that prompts the user belonging to the specific community to indirectly perform behavior modification for solving the issue, in accordance with the control of the intervention section 235. The output device 36 may include broadly IoT devices such as, for example, a smart phone, a tablet terminal, a mobile phone terminal, a PC, a wearable device, a TV, an illumination device, a speaker, and a stand clock.

Having received the command from the information processing apparatus 20, the output device 36 performs an intervention operation in an expression method of the device. For example, when the information processing apparatus 20 transmits the sound and vibration command to the smartphone along with a setting time, the corresponding application on the smartphone sets an alarm at that time. Alternatively, when the information processing apparatus 20 throws a command to the corresponding smart speaker, the speaker plays back music at the specified time. Further, when the information processing apparatus 20 transmits a coupon to the smartphone, a push notification is displayed and the coupon is displayed in a browser. Further, when the information processing apparatus 20 performs the transmission to a PC or the like, the PC may automatically performs conversion into a mail and performs notification to the user. In this manner, the display method is converted into a display method suitable for each output device 36 and the converted display method is outputted, which makes it possible to use any device without depending on a specific model or device.

### [2-2. Operation Process]

Next, processes performed by the respective configurations described above will be described with reference to flowcharts.

### [2-2-1. Overall Flow]

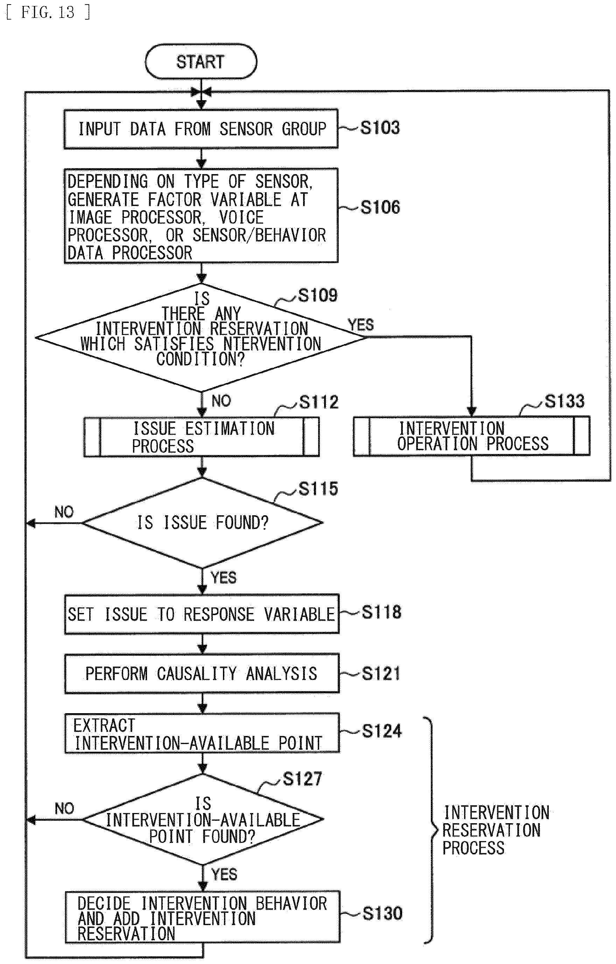

FIG. 13 is a flowchart illustrating an overall flow of an operation process of the first working example. As indicated in FIG. 13, first, the information processing apparatus 20 inputs data from the sensor group (step S103).

Subsequently, depending on the type of the sensor, a factor variable is generated at the image processor 210, the voice processor 212, or the sensor/behavior data processor 214 (step S106).

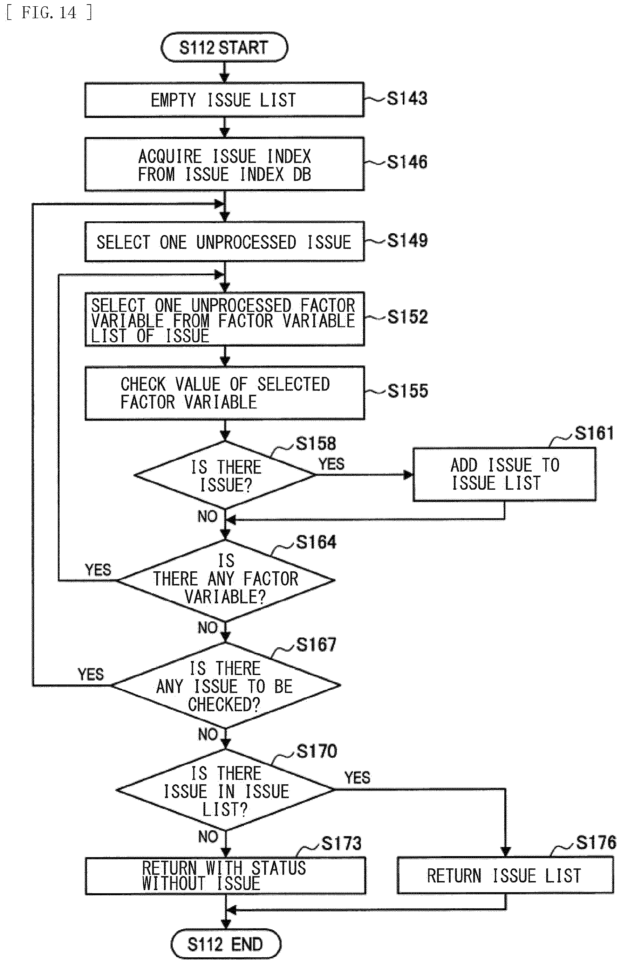

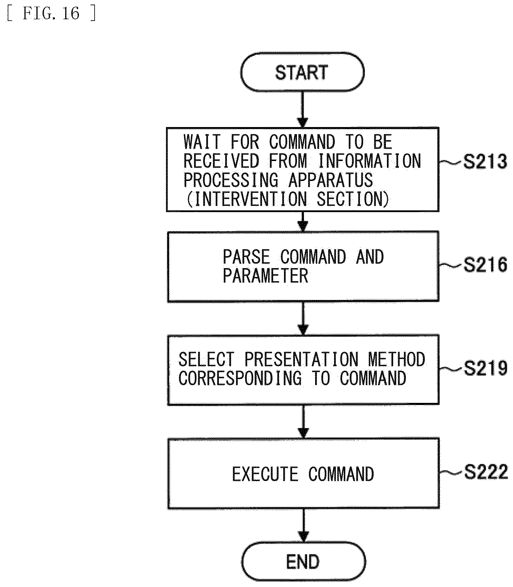

Next, whether or not any intervention reservation which satisfies an intervention condition is present is determined (step S109). If there is none, an issue estimation process is performed (step S112), and if there is such an intervention reservation, an intervention operation process is performed (step S133). It is to be noted that a timing at which step S109 is performed is not limited to this timing, and may be performed in parallel with the process illustrated in FIG. 13. A flow of the issue estimation process is illustrated in FIG. 14. A flow of the intervention operation process is illustrated in FIG. 16.

Subsequently, if an issue is estimated (step S115/Yes), the causality analyzer 232 sets the issue to a response variable (step S118) and performs a causality analysis (step S121).

Next, the intervention section 235 extracts an intervention-available (behavior modification-available) point (causal variable, event) from causal variables included in the causal paths (step S124).

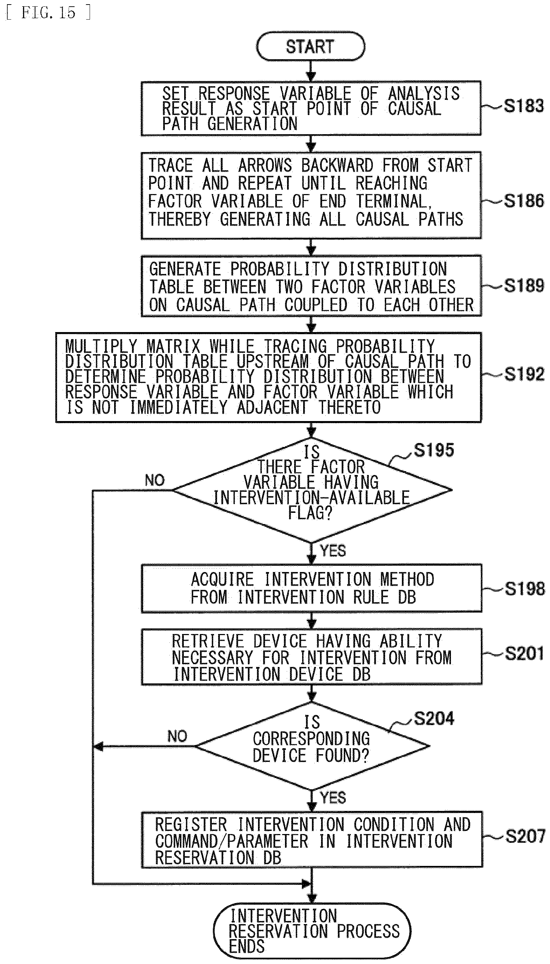

Then, if the intervention-available point is found (step S127/Yes), the intervention section 235 decides an intervention behavior and adds the intervention reservation (step S130). It is to be noted that the process from the extraction of the intervention point to the addition of the intervention reservation will be described in more detail referring to FIG. 15.

### [2-2-2. Issue Estimation Process]

FIG. 14 is a flowchart of the issue estimation process. As indicated in FIG. 14, first, the issue estimation section 230 empties an issue list (step S143) and acquires an issue index (see FIG. 6) from the issue index DB 222 (step S146).

Next, the issue estimation section 230 selects one unprocessed issue from the acquired issue index (step S149).

Thereafter, the issue estimation section 230 selects one unprocessed factor variable from a factor variable list of the issue (see FIG. 6) (step S152), and checks a value of the selected factor variable (step S155). In other words, the issue estimation section 230 determines whether or not the value of the selected factor variable satisfies a condition for determining that there is an issue associated with the issue index (see FIG. 6).

Next, if it is determined that there is an issue (step S158/Yes), the issue estimation section 230 adds the issue to the issue list (step S161).

Thereafter, the issue estimation section 230 repeats steps S152 to S161 described above until the issue estimation section 230 checks all values of the factor variables listed in the factor variable list of the issue (step S164).

In addition, when all issues listed in the issue index have been checked (step S167/No), the issue estimation section 230 returns the issue list (to the causality analyzer 232) (step S176) if there is an issue in the issue list (step S170/Yes). In contrast, if there is no issue in the issue list (step S 170/No), the process returns (to step S115 indicated in FIG. 13) with a status without an issue (step S179).

### [2-2-3. Intervention Reservation Process]

FIG. 15 is a flowchart of the intervention reservation process. As indicated in FIG. 15, first, the intervention section 235 sets a response variable (issue) of the analysis result obtained by the causality analyzer 232 as a start point of the causal path generation (step S183).

Next, the intervention section 235 traces all the arrows backward from the start point and repeats until reaching a factor variable of an end terminal, thereby generating all causal paths (step S186).

Thereafter, the intervention section 235 generates a probability distribution table between two factor variables on a causal path coupled to each other (step S189).

Next, the intervention section 235 multiplies a matrix while tracing the probability distribution table upstream of the causal path to determine a probability distribution between a response variable and a factor variable which is not immediately adjacent to the response variable (step S192).

Thereafter, the intervention section 235 checks if there is a factor variable having the intervention-available flag (step S195) and, if there is a factor variable having the intervention-available flag, the intervention section 235 acquires an intervention method from the intervention rule DB 226 (step S198). It is noted that the intervention section 235 may also determine whether or not to intervene in the factor variable having the intervention-available. For example, in a case where "wake-up time" has the intervention-available flag, and, in order to cause the response variable ("gathering time period") to be within a target range (e.g., "3 hours or more") (to solve the issue that the "gathering time period" is less), the "wake-up time" is to be set to 7:30, the intervention section 235 acquires a user's usual wake-up time trend, and, in a case where the user has a tendency to wake up at 9:00, the intervention section 235 determines that "intervention should be performed" to wake up the user at 7:30.

Next, a device having an ability necessary for the intervention is retrieved from the intervention device DB (step S201), and if the corresponding device is found (step S204/Yes), an intervention condition and a command/parameter are registered in the intervention reservation DB 228 (step S207). For example, if it is possible to control an alarm function of a user's smartphone, an intervention reservation such as "setting the alarm of the user's smartphone at 7:00" is performed.

### [2-2-4. Intervention Process]

FIG. 16 is a flowchart of an intervention process performed by the output device 36. As indicated in FIG. 16, the output device 36 waits for a command to be received from the intervention section 235 of the information processing apparatus 20 (step S213).

Thereafter, the output device 36 parses the received command and parameter (step S216) and selects a presentation method corresponding to the command (step S219).

The output device 36 then executes the command (step S222).

### [2-3. Supplement]

In the first working example described above, the issue related to family closeness is used as an example, but the present embodiment is not limited thereto, and for example, "values gap" may exist as an issue. As a relationship between factors for detecting occurrence of the values gap (estimating an issue), items indicated in Table 5 below may be given, for example.

**[Table 5]**

| Factor variable for determining presence/absence of issue | Condition for determining that issue is present |
|---|---|
| Degree of messiness of room of each family member | Messiness of object placement, types of placed objects Floor exposure of less than 50%, desk exposure of less than 50%, object separation line segment angle variance of less than v, etc. |
| | Whether difference in values for respective room of certain value or more occurs |
| Degree of disagreement | Percentage of agreement with respect to utterance of someone in family of less than 25% |
| Conversation time period taken for decision | Total conversation time period taken for decision of educational policy of child or travel destination, and negative utterance percentage during decision |

The "object separation line segment" in the above Table 5 has a characteristics that, in a case where an image of a messy room is compared to an image of a tidy room, a density of a contour line is low and an individual contour line is long. For this reason, in the image analysis of each user's room or living room, it is possible to calculate the degree of messiness on the basis of, for example, the object separation line segment.

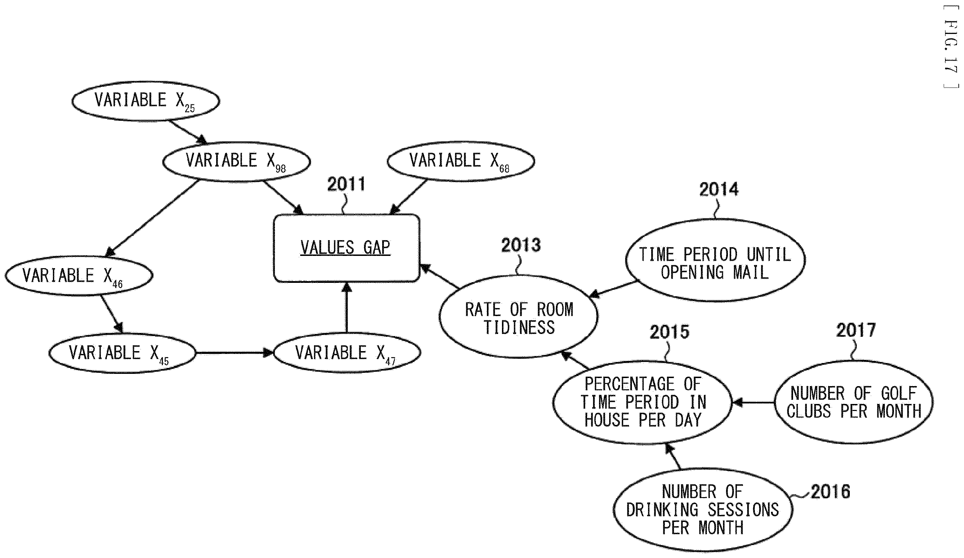

FIG. 17 is a diagram illustrating some examples of causal paths of a values gap. As illustrated in FIG. 17, when causality analysis is performed by setting "values gap" in a response variable 2011, for example, a causal variable 2013 "rate of room tidiness", a causal variable 2014 "time period until opening mail", a causal variable 2015 "percentage of time period in the house per day", a causal variable 2016 "number of drinking sessions per month", and a causal variable 2017 "number of golf clubs per month" rise on the causal paths.

As the intervention methods in this case, there may be given items indicated in Table 6 below, for example.

**[Table 6]**

| Factor variable | Intervention - available flag | Intervention method |
|---|---|---|
| Number of drinking sessions per month | True | Consciously decrease number of drinking sessions, refrain from going to second drinking session |
| Number of golf clubs per month | True | Limit visiting to case of client entertainment that is booked |
| Time period until opening mail | True | Purchase letter opener |
| Rate of room tidiness | False | N/A |

In this way, the intervention section 235 informs the user, for example, to reduce the number of drinking sessions and the number of golf clubs, thereby increasing the time period at home, increasing the "rate of room tidiness" related to the time period at home, and consequently eliminating the family values gap (e.g., a single member having a higher degree of room messiness).

### [3. Second Working Example (Generation of Standard of Value and Behavior Modification)]

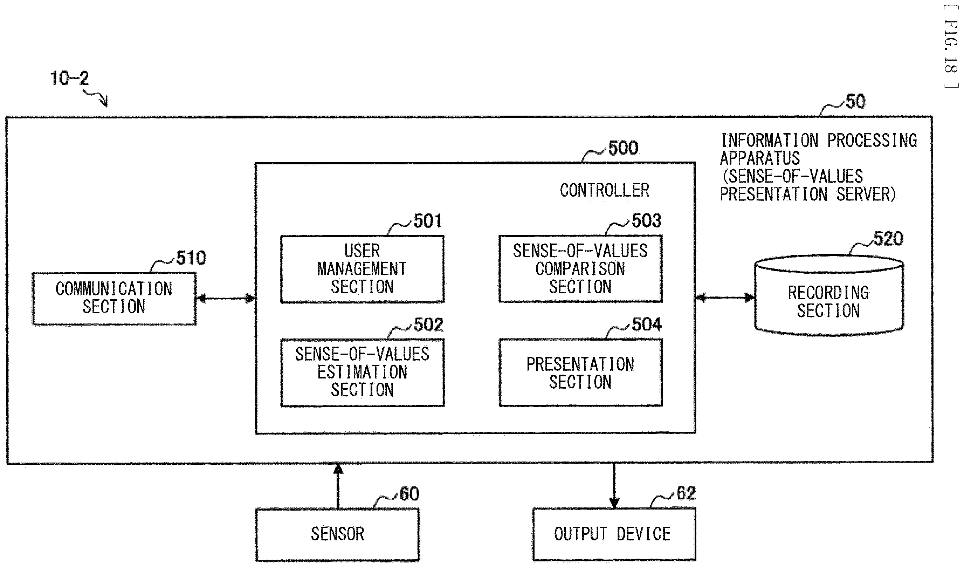

Next, referring to FIGs. 18 to 28, a master system 10-2 according to a second working example will be described.

In the present embodiment, for example, on the basis of data collected from a specific community such as family or a small-scale group (a company, a school, a town association, etc.), a sense of values (standard of value) to be a standard in the community is automatically generated as a behavior rule, and a member who is largely deviated from the standard of value (at a certain degree or more) is indirectly prompted to perform behavior modification (i.e., a behavior to approach the standard of value).

FIG. 18 is a block diagram illustrating an example of a configuration of the master system 10-2 according to the second working example. As illustrated in FIG. 18, the master system 10-2 includes an information processing apparatus 50, a sensor 60 (or a sensor system), and an output device 62 (or an output system).

### [Sensor 60]

The sensor 60 is similar to the sensor group according to the first working example, and is a device/system that acquires every piece of information about the user. For example, environment sensors such as a camera and a microphone installed in a room, and various user sensors such as a motion sensor (an acceleration sensor, a gyroscopic sensor, or a geomagnetic sensor) installed in a smartphone or a wearable device owned by the user, a biometric sensor, a position sensor, a camera, a microphone, and the like, are included. In addition, the user's behavior history (movement history, SNS, shopping history, and the like) may be acquired from the network. The sensor 60 routinely senses behaviors of the members in the specific community and the information processing apparatus 50 collects the sensed behavior.

### [Output Device 62]

The output device 62 is an expressive device that promotes behavior modification, and, similarly to the first working example, includes broadly IoT devices such as, for example, a smart phone, a tablet terminal, a mobile phone terminal, a PC, a wearable device, a TV, an illumination device, a speaker, and a vibrating device.

### [Information Processing Apparatus 50]

The information processing apparatus 50 (sense-of-values presentation server) includes a communication section 510, a controller 500, and a storage 520. The information processing apparatus 50 may be a cloud server on the network, may be an intermediate server or an edge server, may be a dedicated terminal located in a home such as a home agent, or may be an information processing terminal such as a PC or a smartphone.

### -Controller 500

The controller 500 functions as an arithmetic processing unit and a control unit, and controls overall operations in the information processing apparatus 50 in accordance with various programs. The controller 500 is achieved by, for example, an electronic circuit such as CPU (Central Processing Unit) or a microprocessor. Further, the controller 500 may include a ROM (Read Only Memory) that stores programs, operation parameters, and the like to be used, and a RAM (Random Access Memory) that temporarily stores parameters and the like that vary as appropriate.

Further, the controller 500 according to the present embodiment may also function as a user management section 501, a sense-of-values estimation section 502, a sense-of-values comparison section 503, and a presentation section 504.

The user management section 501 manages and stores in the storage 520 as appropriate, information for identifying a user and a sense of values of each user with respect to a target behavior/object. Various indices are assumed for the sense of values, and some examples of the senses of values used in the present embodiment are indicated in Table 7 below. Further, in the present embodiment, a behavior to be sensed (data necessary) for estimating each sense of values may be defined in advance as follows.

**[Table 7]**

| Sense of values | Behavior to be sensed | Sense of values to be standard | information to be recorded |
|---|---|---|---|
| Meal | Observe behavior at meal by camera, etc. | Do not leave food uneaten | Date/time, whether or not food has been left |
| Helping with housework | Observe behavior at meal by camera, etc. | Clear away dishes | Date/time, whether or not dishes have been cleared |
| Aesthetics (desk in office) | Detect number of objects disposed on desk by camera, etc. | Define as standard average number of group | Date/time, number of objects disposed on desk |
| | | | Average of group |
| Aesthetics (child's room) | Detect number of objects scattered on floor using camera, etc., and utterance of mother being angry with child using microphone, etc. | Number of objects scattered on floor when mother is angry (set limit of mother as standard) | Number of objects scattered on floor when mother is angry |
| Childcare | Detect crying volume level at which mother wakes up by baby cry using camera and microphone | Crying volume level at which wife wakes up | Crying volume level at which wife wakes up |
| Object | Measure use frequency and handling of toy using camera image and proximity of radio wave of BLE/RFID, etc. Detect conversation regarding importance of toy using microphone | Level of degree of attachment/affection of child to toy | Register toy with high affection |
| General sense of values | Behavior of base sense of values | Average of group | Base sense of values |

The sense-of-values estimation section 502 automatically estimates (generates) and accumulates in the storage 520 a standard of value (hereinafter, also referred to as standard sense of values) determined in a group (specific community) of the target behavior/object. The sense-of-values estimation section 502 also estimates and manages a sense of values of an individual user. The standard sense of values of the group may be, for example, an average of the senses of values of the respective users in the group (may be calculated by assigning a weight for each members of the group), or a sense of values of a specific user (e.g., parents) may be used as the standard sense of values of the group. What information each sense of values is estimated on the basis of may be defined in advance, for example, as indicated in Table 8 below.

**[Table 8]**

| Sense of values | Estimation of sense of values |
|---|---|
| Meal | Number of times of leaving food, number of times of not leaving food |
| Helping with housework | Number of times of clearing away dishes, number of times of not clearing dishes |
| Aesthetics (desk in office) | Number of objects disposed on desk |
| Aesthetics (child's room) | Number of objects scattered (placed) on floor when mother is angry |
| Childcare | Crying volume level at which mother wakes up |
| Obj ect | Level of degree of attachment/affection of child to toy |
| General sense of values | Estimate based on value obtained by normalizing base sense of values |

The sense-of-values comparison section 503 detects deviation of the sense of values of each user from the standard sense of values of the behavior/object that is routinely sensed. The standard sense of values of the group may be automatically generated by the sense-of-values estimation section 502 as described above, or may be preset (defaults may be set on the system or manually set by the user of the group).

The presentation section 504 promotes, in a case where the deviation occurs in the sense of values, the behavior modification for causing the sense of values to approach the standard sense of values of groups. Specifically, the presentation section 504 transmits a behavior modification command from the communication section 510 to the output device 62.

### [Communication Section 510]

The communication section 510 is coupled via wire or radio to external devices such as the sensor 60 and the output device 62, and transmits and receives data. The communication section 510 communicates with the external devices by, for example, a wired/wireless LAN (Local Area Network), or Wi-Fi (registered trademark), Bluetooth (registered trademark), a mobile communication network (LTE (Long Term Evolution), 3G (third-generation mobile communication system)), or the like.

### [Storage 520]

The storage 520 is achieved by a ROM (Read Only Memory) that stores programs, operation parameters, and the like to be used for the processing performed by the controller 500, and a RAM (Random Access Memory) that temporarily stores parameters and the like that vary as appropriate.

The configuration of the master system 10-2 according to the present embodiment has been described in detail above.

### [3-2. Operation Process]

Subsequently, an operation process of the master system 10-2 described above will be described with reference to flowcharts.

### (3-2-1. Basic flow)

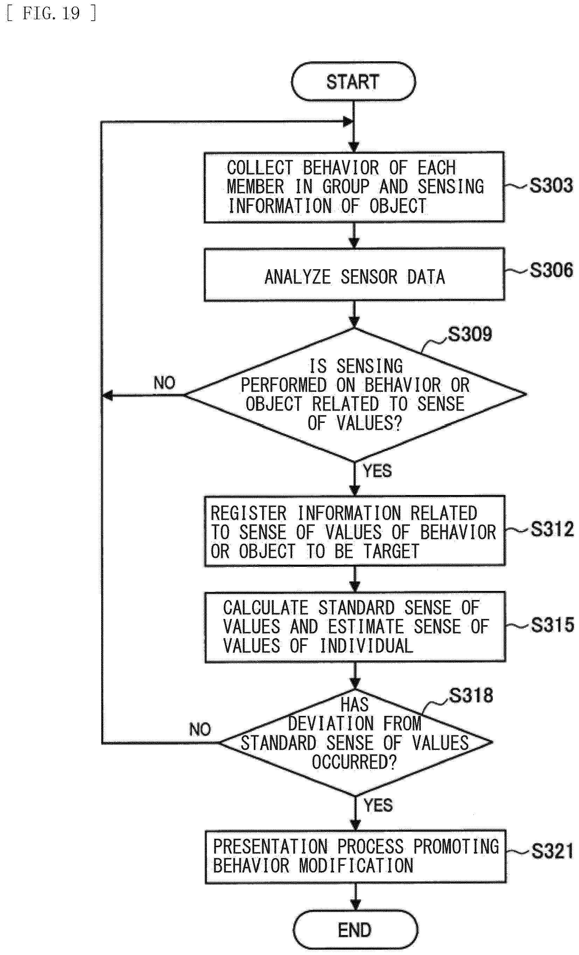

FIG. 19 is a basic flowchart of an operation process according to the present embodiment. As illustrated in FIG. 19, the information processing apparatus 50 first collects (step S303) and analyzes (step S306) a behavior of each member in a group and sensing information of an object.

Next, in a case where it is possible to perform sensing on the behavior or the object related to the sense of values (step S309/Yes), the information processing apparatus 50 registers information related to the sense of values of the behavior or the object to be a target (step S312).

Thereafter, the information processing apparatus 50 performs calculation of the standard sense of values (of the group) and estimation of a sense of values of an individual (individual sense of values) (step S315). The standard sense of values may be calculated, for example, by averaging the senses of values of the respective members of the group, or by using a sense of values of someone in the members as the standard sense of values.

Subsequently, in a case where a member deviates from the standard sense of values (step S318/Yes), the information processing apparatus 50 performs a presentation process for prompting the member to perform behavior modification (step S321). For example, in a case where the sense of values (individual sense of values) of the member deviates from the standard sense of values (of the group), predetermined UI presentation or the like for promoting the behavior modification is performed. Such information presentation for promoting the behavior modification may be presentation of a specific instruction for eliminating the deviation from the standard sense of values, or presentation of content for promoting casually the behavior modification.

The basic flow described above will be described below using a specific examples. Hereinafter, information presentation processes of the behavior modification will be described in detail using specific examples of the sense of values.

### [3-2-2. Sense of Values Related to Meal]

First, "meal discipline" is assumed as a sense of values related to "meal", that is, food is valued and meal is not to be left uneaten. In the present embodiment, in a case of deviating from such a sense of values of the meal, presentation is performed for prompting a member of the target to perform behavior modification.

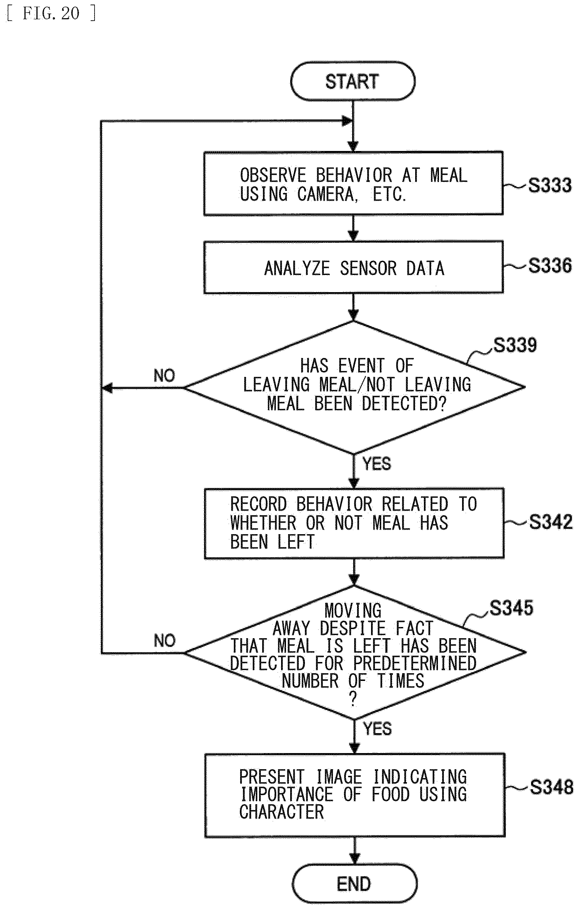

FIG. 20 is a flowchart illustrating a behavior modification process related to the meal discipline according to the present embodiment. As illustrated in FIG. 20, first, the information processing apparatus 50 observes a behavior of a meal using a camera or the like (step S333), and analyzes sensor data (step S336).

Next, if an event of leaving the meal/not leaving the meal is detected (step S339/Yes), the information processing apparatus 50 records the behavior related to whether or not the meal has been left (step S342).

Next, if the information processing apparatus 50 detects that a member has moved away despite a fact that the meal is left a predetermined number of times (step S345/Yes), information presentation for prompting the member not to leave the meal is performed. For example, for a child, an image indicating importance of food (rice and vegetables) by using a character is presented. In the present embodiment, the number of times of meals left is estimated as the individual sense of values, and if a behavior that differs from a sense of values of the majority of the group as a whole (a standard sense of values of a group to be a behavior rule, e.g., in a case where the majority of the group do not leave the meal every time, it is the standard sense of values of this group to not leave the meal every time) is performed a predetermined number of times, it is determined that a deviation from the standard sense of values has occurred. The image may be presented on a smartphone or a wearable device of a target child, or may be projected onto a table by a projector. In addition, it may be outputted by sound such as sound AR where the remaining food is heard as if the food is speaking, such as "don't leave me!" or "one grain is worth thousand grains".

### [3-2-3. Sense of Values Related to Housework]

In addition, assumed as a sense of values related to housework is, for example, "after meals, all family members clear away dishes". In a case of deviating from such a sense of values of housework, presentation is performed for prompting a member of the target to perform behavior modification.

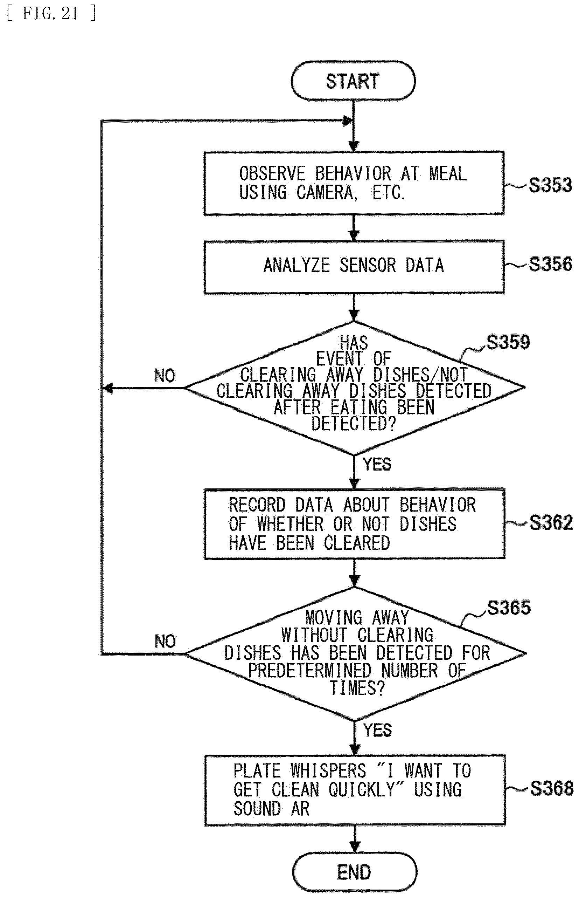

FIG. 21 is a flowchart illustrating a behavior modification process related to the clearing away of dishes according to the present embodiment. As illustrated in FIG. 21, first, the information processing apparatus 50 observes a behavior of a meal using a camera or the like (step S353), and analyzes sensor data (step S356).

Next, if an event of clearing away dishes/not clearing away dishes is detected (step S359/Yes), the information processing apparatus 50 records behavior data of whether each member has cleared away dishes (step S362).

Next, if the information processing apparatus 50 detects a fact that a member has moved away without clearing dishes predetermined number of times (step S365/Yes), the information processing apparatus 50 may present information promoting dish clearing, e.g., for a child, may output a voice that a plate whispering "I want to get cleaned quickly" using sound AR. In the present embodiment, the number of times of dishes cleared away is estimated as the individual sense of values, and if a behavior that differs from a sense of values of the majority of the group as a whole (a standard sense of values of a group to be a behavior rule, e.g., in a case where the majority of the group clear away dishes every time, it is the standard sense of values of this group to clear away dishes every time) is performed a predetermined number of times, it is determined that a deviation from the standard sense of values has occurred.

### [3-2-4. Aesthetics of Room]

Further, assumed as a sense of values of an aesthetics of a room such as an office or an own room is, for example, a degree of tidiness (a degree of clearance), such as a fact that objects are not scattered on a floor or a desk.

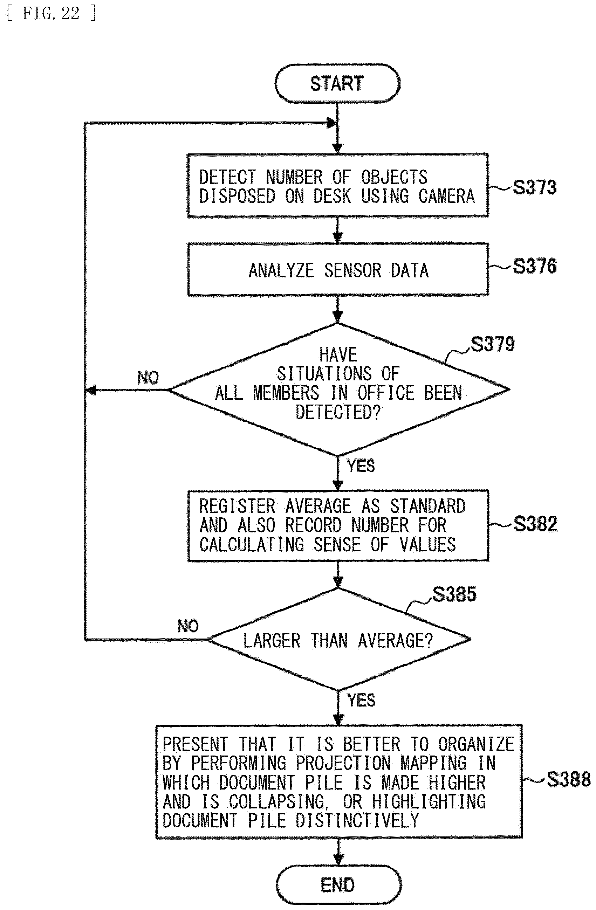

FIG. 22 is a flowchart of a behavior modification process related to clearing up of a desk in an office. As illustrated in FIG. 22, for example, the information processing apparatus 50 detects (captures) the number of objects disposed on the desk of the office using a camera or the like (step S373), and performs an analysis (calculation of the number of objects by an image analysis or the like) of sensor data (step S376). Although "the number of objects" is used as an example here, the degree of tidiness may be detected by the image analysis.

Next, if situations of all members in the office are detected (step S379), the information processing apparatus 50 registers the average number of a group as a standard (a standard sense of values), and also records the number of objects disposed on the desk of each member for an individual sense of values calculation (step S382).

Thereafter, if the number of objects on a desk of a member is larger than the average of the group (step S385/Yes), the information processing apparatus 50 may indirectly indicate that it is better to organize the desk by performing information presentation prompting the member to clear up the desk, e.g. by projecting projection mapping in which a document pile is made higher and is collapsing, or highlighting document pile distinctively. The information presentation prompting the member to clear up the desk may be presented by sound, such as sound AR.

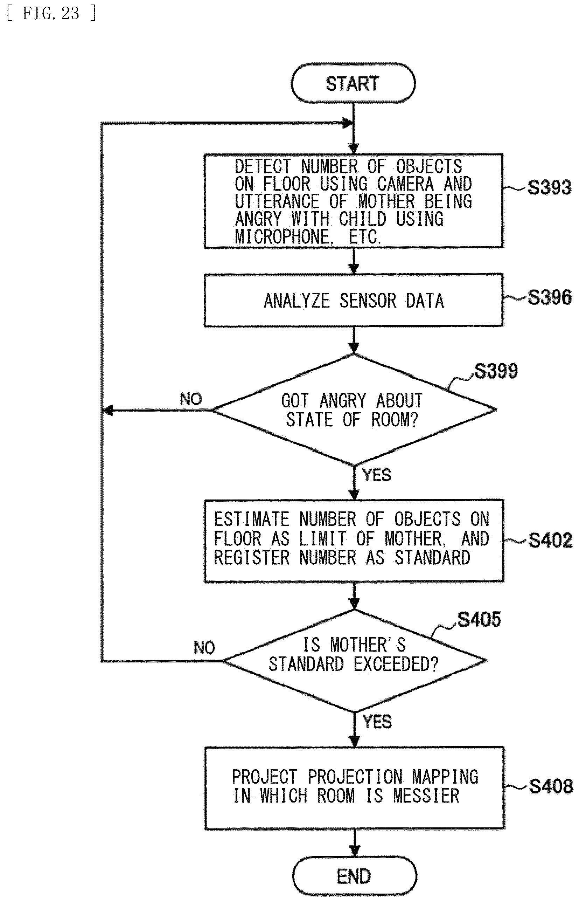

FIG. 23 is a flowchart of a behavior modification process related to tidying up of a room. As illustrated in FIG. 23, first, the information processing apparatus 50 detects the number of objects scattered (lying) on the floor using a camera or the like and an utterance of a mother being angry with a child using a microphone or the like (step S393), and analyzes the sensor data (step S396).

Next, if the mother is angry at the child about the room state (step S399/Yes), the information processing apparatus 50 considers the number of objects lying on the floor as a limit of the mother and registers the number as the standard sense of values of a group (step S402). In the present embodiment, regarding the aesthetics of the room, the limit of the mother is defined as the standard of value of the group. It is to be noted that the aesthetics of the room is not limited to the number of objects lying on the floor, and for example, the standard of value may be defined on the basis of a sensing target such as a percentage of a floor area of the room (a situation where there is nowhere to step a foot on can be said that the situation is messy), a difference from a state of a usual room (a floor area, a degree of tidiness, etc.), or the like.

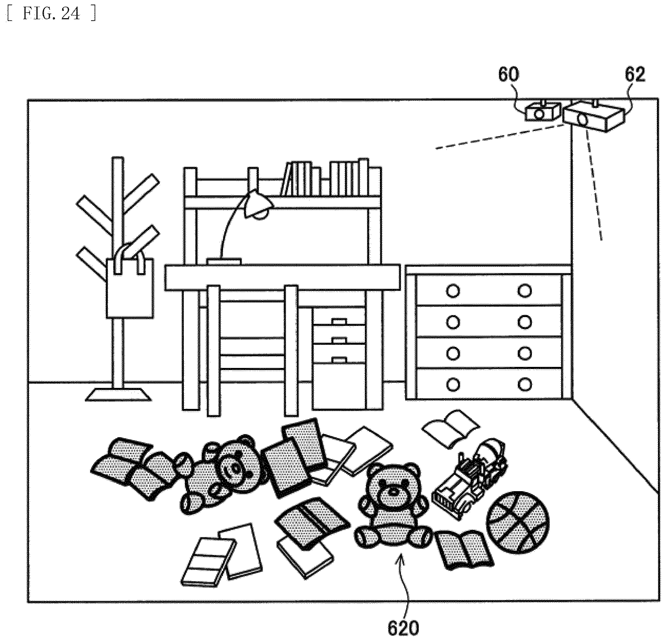

Thereafter, if the situation of the room exceeds the mother's standard (i.e., if the number of objects lying in the room exceeds "the number of objects" of the standard sense of values of the group, which is to be the mother's standard) (step S405/Yes), information presentation promoting tidying up of the room is performed, e.g., projecting, as illustrated in FIG. 24, a projection mapping in which the room is messier. In the case illustrated in FIG. 24, the number of objects lying on the floor is detected by the sensor 60 such as a camera installed in the room, and, if the number of objects lying on the floor exceeds the standard, an image 620 which looks messier is projected by the projection mapping by the output device 62 such as a projector. The information presentation promoting tidying up of the room may be presented by sound, such as sound AR.

Further, the parents may be presented with an image of a situation of the room from a current child's point of view. It is also possible to map the degree of messiness of the room to other values, such as emotions, to be presented to the child. For example, when the room is dirty, a hero associated with the room is weakened or becomes bad looking.

### [3-2-5. Sense of Values Related to Childcare]

In addition, regarding a sense of values related to childcare, for example, the mother notices at once about night cry of a baby, but the father is generally slow to respond. Thus, the following is given as an example: an acceptable level of the mother with respect to the baby cry (must wake up and cuddle the baby) is defined as the standard sense of values of the group and the father's behavior modification is promoted.

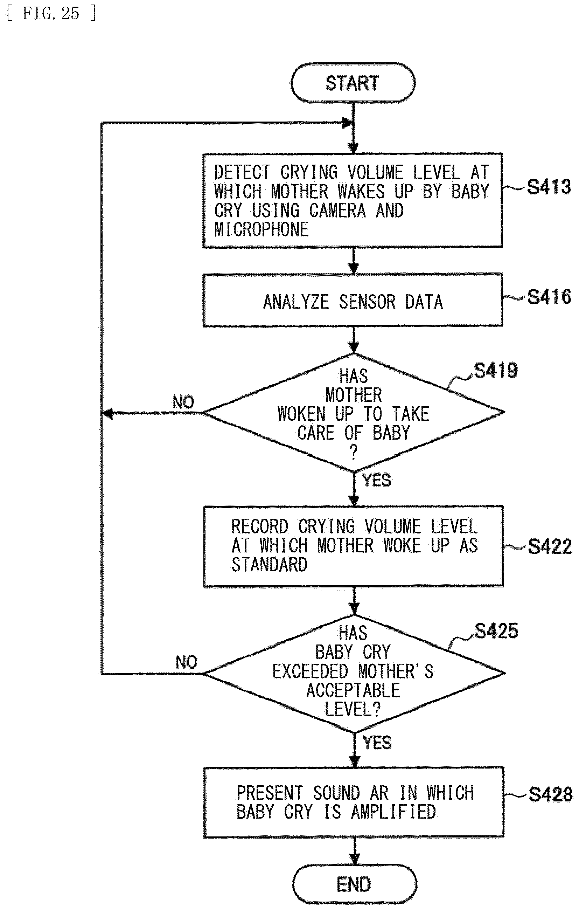

FIG. 25 is a flowchart of a behavior modification process related to baby cry. As illustrated in FIG. 25, for example, the information processing apparatus 50 detects a crying volume level when the mother wakes up by the baby cry by using a camera, a microphone, or the like (step S413), and analyzes the sensor data (step S416).

Next, if the mother wakes up to take care of the baby (step S419/Yes), the information processing apparatus 50 registers the crying volume level at which the mother woke up as a standard (which is the mother's acceptable level and is set as the standard sense of values of the group) (step S422).

Thereafter, if the baby cry exceeds the acceptable level of the wife (i.e. standard sense of values of the group) (step S425/Yes), the information processing apparatus 50 performs information presentation prompting the father to wake up, e.g. presents sound AR in which the baby cry is amplified to the father (step S428).

### [3-2-6. Sense of Values toward Object]

In addition, regarding the sense of values related to affection toward an object, for example, a certain specific stuffed toy is extremely important for a child, but the mother treats all stuffed toys in the same manner. In the present embodiment, in a case where a difference between the sense of values of the child and the sense of values of the mother with respect to the object (e.g., a stuffed toy) become greater than or equal to a certain value, it becomes possible to visualize the sense of values of the child and prompt the father to (indirectly) perform behavior modification.

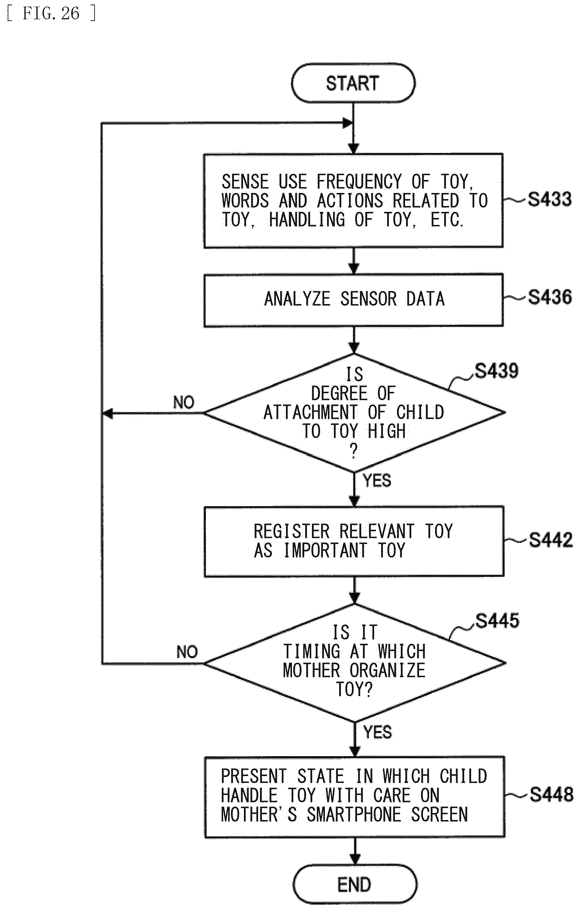

FIG. 26 is a flowchart illustrating a behavior modification process related to a toy. As illustrated in FIG. 26, first, the information processing apparatus 50 senses a use frequency of the toy, words and actions related to the toy, handling of the toy, and the like, by using a camera, a microphones, or the like (step S433), and analyzes the sensor data (step S436). Specifically, for example, it is possible to measure the use frequency (the frequency at which the child plays with the toy) by using a camera image or proximity of a radio wave such as BLE/RFID (transmitted from the toy). The microphone may also be used to collect conversations and extract and count utterances about which toy is important, which toy is fun to play with, which toy is his/her favorite, etc. In addition, it is also possible to measure the handling of the toy (whether the handling is careful or rough) by using an image captured by a camera, a voice of a conversation from a microphone, radio waves such as BLE/RFID, and the like.

Next, if a degree of attachment (e.g., a degree of affection) of the child to the toy is high (step S439/Yes), the information processing apparatus 50 registers the toy as an important toy (a toy having a high degree of affection) (step S442).

Next, if it is a timing at which the mother is to organize toys (step S445/Yes), for example, in a case where a toy that the mother is trying to discard is the toy having a high degree of affection of the child, information of the sense of values of the child is presented to the mother, for example, an image that the child handles the toy with care is presented on the mother's smartphone or the like (step S448). It is possible to determine the timing at which the mother organizes the toys, for example, by analyzing an image captured by a camera or by analyzing a sound collected by the microphone (utterances such as "there are too many toys, so I'm going to organize them" or "I'm going to discard them"). In addition, it is possible to determine which toy the mother is attempting to discard on the basis of, for example, an analysis of an image captured by a camera, an analysis of radio waves transmitted from a tag such as a BLE/RFID provided on the toy (which becomes undetectable due to a fact that the toy is discarded to a trash box or the like), or the like.

### [3-2-7. General Sense of Values]

Next, assumed as an example of the sense of values is, a sense of values of what kind of sense of values is regarded to be important (a general sense of values). The sense of values (a base sense of values) used as a base when calculating the general sense of values includes, for example, the above-mentioned "value meals", "all family members help with housework", "aesthetics (room tidiness state)", "childcare", "affection toward object", and the like. On the basis of those base senses of values, a sense of values (i.e., "general sense of values") as to which sense of values (to be a candidate of the general sense of values) each member attaches an importance is estimated and, for example, an average of the group is taken as a general sense of values. Thereafter, if a deviation occurs between the general sense of values of the group and the general sense of values of the individual (member), it is possible to prompt the member to perform behavior modification (e.g., to adjust the general sense of values of the group) by presenting the general sense of values of the group to the member.

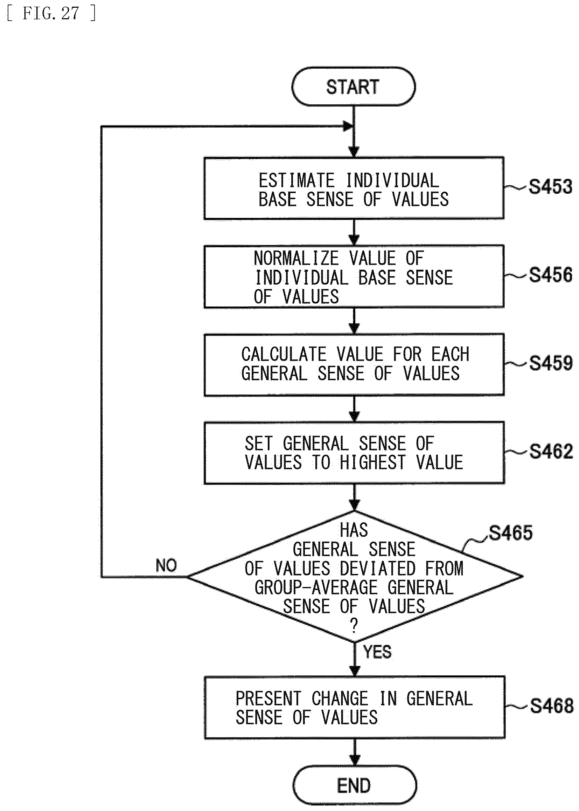

FIG. 27 is a flowchart showing a behavior modification process related to the general sense of values. As illustrated in FIG. 27, the information processing apparatus 50 first estimates the base sense of values of the individual (each members of the group) (step S453).

Next, the information processing apparatus 50 normalizes a value of the base sense of values of the individual (step S456).

Thereafter, the information processing apparatus 50 refers to a sense of values association table, and calculates a value for each general sense of values in accordance with a weighted value of the associated general sense of values (step S459). Here, an example of the sense of values association table is indicated in Table 9 below. As indicated in Table 9 below, examples of the candidates of the general sense of values include "honesty", "caring", "society", and "individuality".

**[Table 9]**

| Base sense of values | Corresponding general sense of values |
|---|---|
| Value meals | Honesty 20%, caring 10% |
| All family members help with housework | Honesty 10%, caring 20%, society 30% |
| Aesthetics (case of child's room) | Honesty 10%, caring 10%, society 40% |
| Childcare | Caring 50% |
| Affection toward object | Individuality 30%, caring 10% |

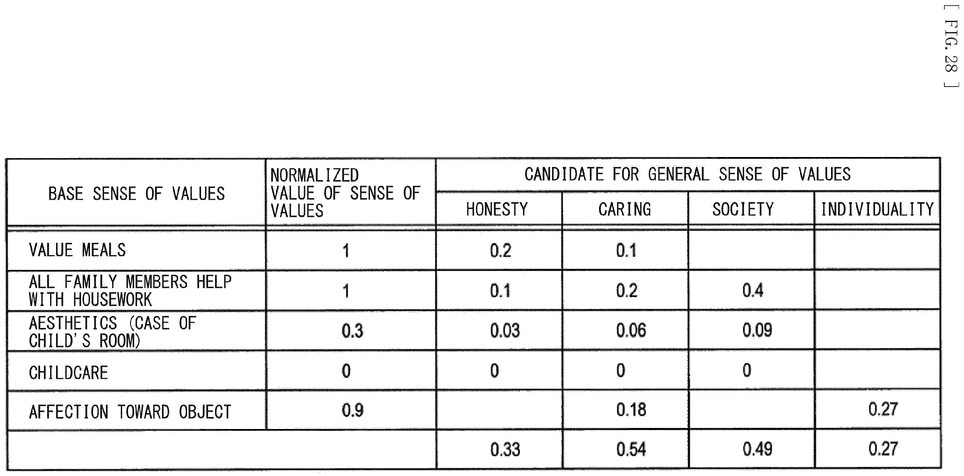

Next, the information processing apparatus 50 sets the general sense of values to the highest value (i.e., the most important sense of values) (step S462). Here, FIG. 28 illustrates an example of values for each general sense of values of an individual member calculated by referring to the weights indicated in Table 9. In the case illustrated in FIG. 28, since the value of the sense of values of "caring" has the highest value, this sense of values is a sense of values that the member regards as the most important value, that is, the "general sense of values" of the member.

Thereafter, if the general sense of values of the member deviates from the group-average general sense of values (step S465/Yes), the information processing apparatus 50 presents the change in the general sense of values to the member (step S468).

### [4. Third Working Example (Adjustment of Life Rhythm)]

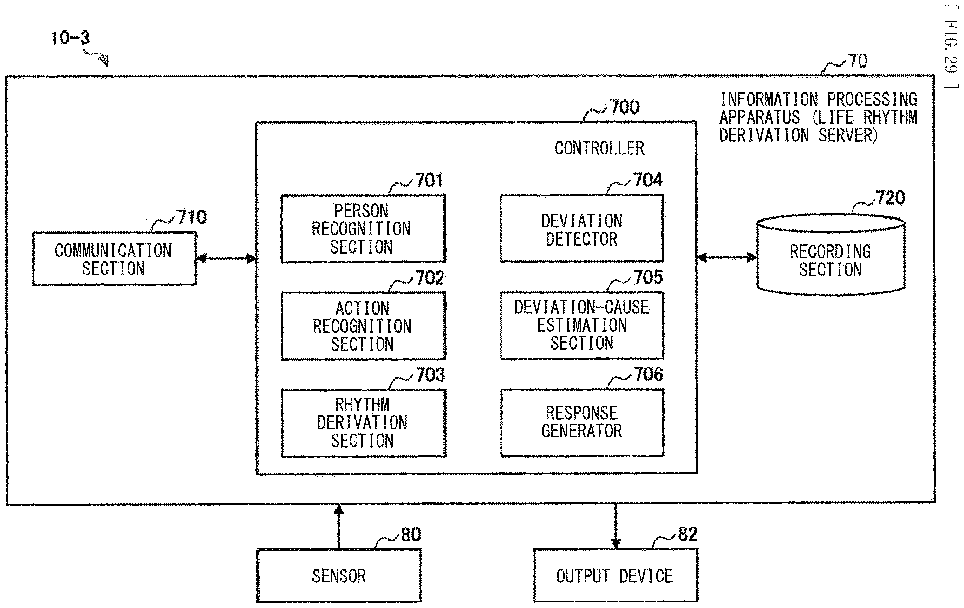

Next, referring to FIGs. 29 to 35, a master system 10-3 according to a third working example will be described.

In the present embodiment, for example, when an issue that a gathering time period is insufficient is estimated on the basis of data collected from a family (the estimation of the issue is similar as the first working example), it is to adjust a meal time slot that is set to a behavior rule to solve the issue, a life rhythm of the family (an evening meal time slot of each member or the like) is detected, and behavior modification of the life rhythm is indirectly promoted to cause the meal times of the respective members to be adjusted.

FIG. 29 is a block diagram illustrating an example of a configuration of the master system 10-3 according to the third working example. As illustrated in FIG. 29, the master system 10-3 includes an information processing apparatus 70, a sensor 80 (or a sensor system), and an output device 82 (or an output system).

### [Sensor 80]

The sensor 80 is similar to the sensor group according to the first working example, and is a device/system that acquires every piece of information about the user. For example, environment sensors such as a camera and a microphone installed in a room, and various user sensors such as a motion sensor (an acceleration sensor, a gyroscopic sensor, or a geomagnetic sensor) installed in a smartphone or a wearable device owned by the user, a biometric sensor, a position sensor, a camera, a microphone, and the like, are included. In addition, the user's behavior history (movement history, SNS, shopping history, and the like) may be acquired from the network. The sensor 60 routinely senses behaviors of the members in the specific community and the information processing apparatus 70 collects the sensed behavior.

### [Output Device 82]

The output device 82 is an expressive device that promotes behavior modification, and, similarly to the first working example, includes broadly IoT devices such as, for example, a smart phone, a tablet terminal, a mobile phone terminal, a PC, a wearable device, a TV, an illumination device, a speaker, and a vibrating device.

### [Information Processing Apparatus 70]

The information processing apparatus 70 (life rhythm derivation server) includes a communication section 710, a controller 700, and a storage 720. The information processing apparatus 70 may be a cloud server on the network, may be an intermediate server or an edge server, may be a dedicated terminal located in a home such as a home agent, or may be an information processing terminal such as a PC or a smartphone.

### -Controller 700

The controller 700 functions as an arithmetic processing unit and a control unit, and controls overall operations in the information processing apparatus 70 in accordance with various programs. The controller 700 is achieved by, for example, an electronic circuit such as CPU (Central Processing Unit) or a microprocessor. Further, the controller 700 may include a ROM (Read Only Memory) that stores programs, operation parameters, and the like to be used, and a RAM (Random Access Memory) that temporarily stores parameters and the like that vary as appropriate.

Further, the controller 700 according to the present embodiment also functions as a person recognition section 701, an action recognition section 702, a rhythm derivation section 703, a deviation detector 704, a deviation-cause estimation section 705, and a response generator 706.

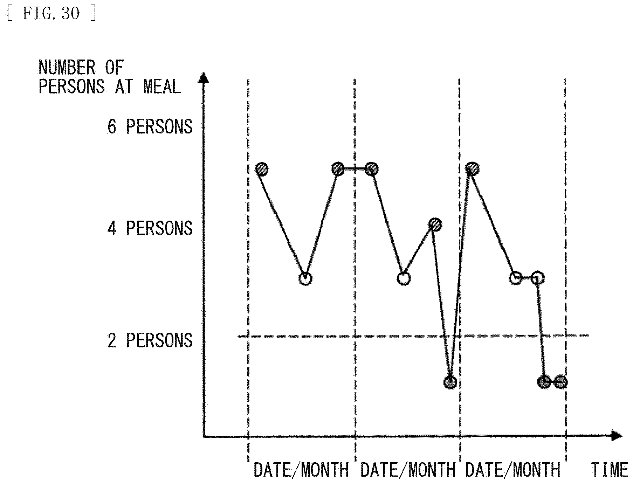

The person recognition section 701 recognizes a person by performing facial recognition or the like on an image captured by a camera. The action recognition section 702 recognizes an action of each user (e.g., returning home, eating, bathing, relaxing time, sleeping, etc.) on the basis of an image captured by a camera and various pieces of sensor data. More specifically, for example, a sensor at home (a camera, a microphone, or the like) senses a home returning time, a meal time, etc., of the family, and records the home returning time, the time at which the meal is taken, etc. In addition, as illustrated in FIG. 30, a person with whom the meal is taken, the number of people who have eaten together, and the like are also recorded.

The rhythm derivation section 703 calculates, on the basis of the above-mentioned behavior record of the family, the life rhythm of the family (e.g., trends of the home returning time, the meal time, the bathing time, etc. for each day of the week).

The deviation detector 704 compares the life rhythm of the respective members of the family to each other and detects a deviated portion. For example, if a frequency that only a father's evening meal time deviates significantly from a rhythm of the evening meal time of the family increases, a deviation is detected.

The deviation-cause estimation section 705 estimates a causes of the deviation. For example, if the frequency that only the evening meal time of the father greatly deviates increases, the cause of the deviation of the evening meal time of the father is estimated. For the estimation of the cause, there is given, for example, a method of causal analysis or a method using Bayesian estimation. For example, in a case where a family member is unable to take the evening meal with the family because the home returning time is late on Thursday every week, it is possible to estimate that the home returning time being late is caused by a regular meeting at work on Thursday every week.

The response generator 706 generates a response that indirectly promotes behavior modification to adjust the life rhythm. For example, as described above, in a case where only the father is often unable to take the evening meal together on Thursday, it has been possible to analyze that the cause is the regular meeting, and therefore, advice such as "how about changing the Thursday regular meeting?" is presented to the father from the PC screen or the like. Following this advice makes it possible as a result to take the evening meal with the family. It is to be noted that the advice may be presented using an e-mail, an SNS, or another messaging function.

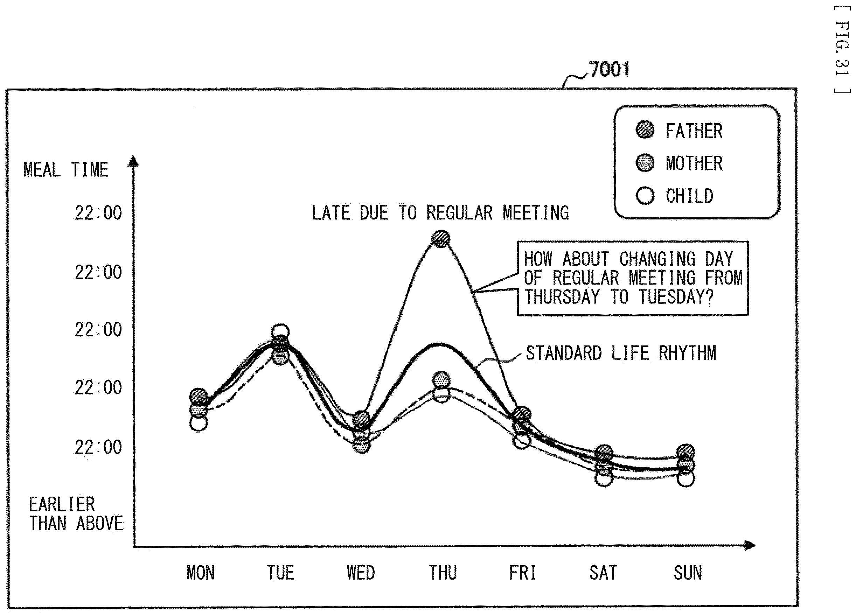

Here, FIG. 31 illustrates a diagram for explaining a deviation in a life rhythm. FIG. 31 illustrates evening meal times of the father, the mother, and the child, and a life rhythm to be a standard of the family (behavior rule). The life rhythm to be the standard of the family is, for example, an accumulated average time of evening meal times of the respective family members on each day of the week. As illustrated in FIG. 31, in a case where the life rhythms of the evening meal times of the father, the mother, and the child are calculated, it can be appreciated that only the meal time of the father on Thursday has a large deviation. In this case, it can be appreciated that, on the basis of the cause estimation, the home returning time is late due to the regular meeting, for example, and the meal time is shifted. In contrast, it can be appreciated that evening meal time period of the family on Tuesday is late compared to the other days of the week. In this case, by changing the day on which the regular meeting is held to Tuesday, the father is in time for the early meal time on Thursday, and also, there is a possibility that the father may have the evening meal together on Tuesday even if the home returning time is late due to the regular meeting, because the meal time on Tuesday is late. Accordingly, the response generator 706 is able to generate concrete advice such as "how about changing the day of the regular meeting from Thursday to Tuesday?" It is to be noted that an image 7001 including the graph and the advice illustrated in FIG. 31 may be presented to the father as advice.

### [Communication Section 710]

The communication section 710 is coupled via wire or radio to external devices such as the sensor 80 and the output device 82, and transmits and receives data. The communication section 710 communicates with the external devices by, for example, a wired/wireless LAN (Local Area Network), or Wi-Fi (registered trademark), Bluetooth (registered trademark), a mobile communication network (LTE (Long Term Evolution), 3G (third-generation mobile communication system)), or the like.

### [Storage 720]

The storage 720 is achieved by a ROM (Read Only Memory) that stores programs, operation parameters, and the like to be used for the processing performed by the controller 700, and a RAM (Random Access Memory) that temporarily stores parameters and the like that vary as appropriate.

The configuration of the master system 10-3 according to the present embodiment has been described above in detail.

### [4-2. Operation Process]

Next, an operation process performed by the master system 10-3 described above will be described referring to a flowchart.

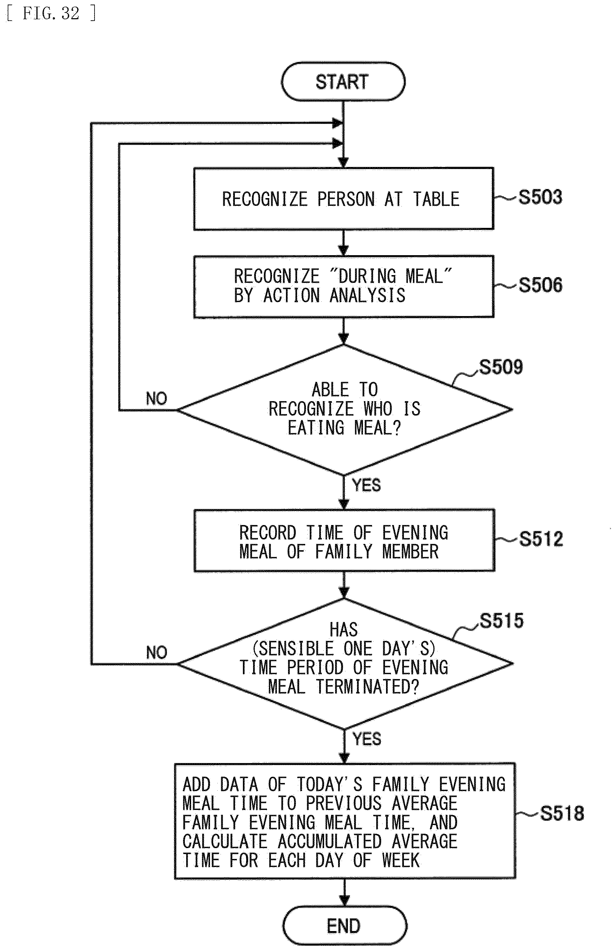

FIG. 32 is a flowchart of an operation process of generating a rhythm of an evening meal time. As illustrated in FIG. 32, first, the information processing apparatus 70 recognizes a person at a dining table by a camera or the like (step S503), and recognizes that the person is "during meal" by an action analysis (step S506).

Next, if it is possible to recognize who is eating the meal (step S509/Yes), the information processing apparatus 70 records the time of the evening meal of the family member (step S512). An example of the record of the evening meal times of the members of the family is indicated in Table 10 below.

**[Table 10]**

| Person ID | Evening meal time |
|---|---|
| 00011 (father) | 20:30, Thu., 26/09/2017 |
| 00012 (mother) | 18:30, Thu., 26/09/2017 |
| 00013 (child) | 17:30, Thu., 26/09/2017 |

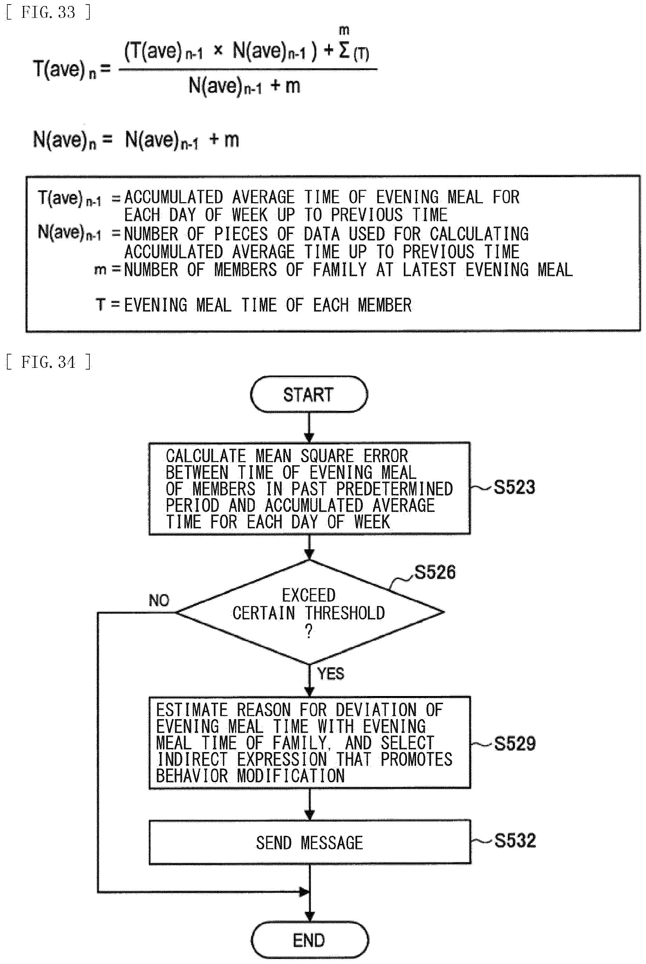

Thereafter, if a (sensible one day's) time period of the evening meal has terminated (step S515/Yes), the information processing apparatus 50 adds data of today's family evening meal time to previous average family evening meal time, and calculates accumulated average time for each day of the week (generates evening meal time rhythm) (step S518). Here, an example of a calculation formula of the accumulated average time for each day of the week is indicated in FIG. 33. For example, the accumulated average time for each day of the week, that is, a life rhythm to be the standard of the family illustrated in FIG. 31 may be calculated on the basis of the calculation formula indicated in FIG. 33.

FIG. 34 is a flowchart for generating advice on the basis of the life rhythm. As illustrated in FIG. 34, the information processing apparatus 70 first detects a deviation of the life rhythm of the family. More specifically, for example, the information processing apparatus 70 calculates the mean square error between the time of evening meal of members in a past predetermined period (for example, three months) and the accumulated average time for each day of the week (step S523).

Next, if the calculated error exceeds a predetermined threshold (step S526/Yes), the information processing apparatus 70 estimates a reason that the evening meal time deviates from the evening meal time of the family (a cause of the deviation), and selects an indirect expression that promotes the behavior modification (step S529). The estimation of the cause of the deviation may be performed by, for example, a method of causal data analysis.

Thereafter, the information processing apparatus 70 sends a message in the selected indirect expression (step S532).

It is to be noted that that behavior modification for adjusting the time of the evening meal is not limited to prompting only the father who has deviation in the time of the evening meal, but may be prompting other family members so that the evening meal may be taken with all of the family members as a result. For example, the life rhythm of the family is modified such that, for example, the family members are in the vicinity of a nearest station at the home returning time of the father. Specifically, for example, on the basis of the life rhythm illustrated in FIG. 31, the master system 10-3 advises the mother on Thursday evening, saying, "how about going out with your child to the station", "it seems that the shop XX in front of the station is popular", or the like. When the mother shops with her child in front of the station in accordance with her master's words, the father on the way home contacts her telling, "I'm almost at the station." The mother replies "oh, I'm just near the station", and the family members naturally join together, which makes it possible to for them to have an evening meal at a restaurant near the station.

In addition, although the "evening meal time" is exemplified as the life rhythm in the present embodiment, the present disclosure is not limited thereto, and for example, also assumed are a wake-up time, a sleeping time, a working time, an exercise time, a media viewing time, and the like.

### [4-3. Modification Example]

In the above-described embodiment, it has been desired to adjust the life rhythms, however, the present disclosure is not limited thereto. For example, indirect advice may be provided to cause the life rhythms to be deviated from each other (asynchronous). For example, it is preferable that a bathing time, a toilet time, a time to use the washbasin, and the like be deviated from each other.

The information processing apparatus 70 has a knowledge table of events or the like that occur when the life rhythms are synchronized, as indicated in Table 11 below, and, by referring to the table below, indirectly provides advice to perform behavior modification of shifting the life rhythms of the community members.

**[Table 11]**

| Type of behavior (Life rhythm) | Event that may occur when behaviors are synchronized | Threshold of overlapping | Advice |
|---|---|---|---|
| Wake-up time | Concentration of people in toilet | Two persons or more | Adjust snooze of alarm clock of person with low degree of urgency (may be predicted from schedule, may be estimated based on on/off of work or |
| | | | school, or may be determined based on priority setting or the like of alarm) to increase length, or issue notification of "you'd better move your wake-up time." |
| TV-viewing time after evening meal | Concentration of people in bathroom | Three persons or more | Issue notification that you can stay in bathtub for long if you go in now (to person who is not watching TV, person who seems to be not interested in TV, person who takes long bath, person who likes bath, etc.) |
| Breakfast-start time | Concentration of people in washroom | Three persons or more | Issue notification that you can use washroom now to person who has finished breakfast, person who takes long time for getting prepared, person who has to go out early, etc. |

Assume that, for example, the master system 10-3 routinely senses behaviors of the family members by a sensor such as a camera or a microphone installed at home, and records situations. In this case, for example, in a case where the master system 10-3 detects a situation that the majority of the family members (the father, the mother, a daughter, etc.) gather in a living room and watch a television, and knows that the bathroom tends to be crowded every time after the television is watched (it may be learned and acquired, or may be registered in advance, see Table 11), the master system 10-3 notifies the eldest son, who is alone in his room without watching the television, that "you can use the bathtub for a long time if you go in now". The notification may be issued by various output devices such as a smartphone, a projector, a speaker, and the like. The eldest son who has been studying in his room may say "good timing, I wanted to relax in the bathtub," and is able to temporarily stop studying and take a bath.

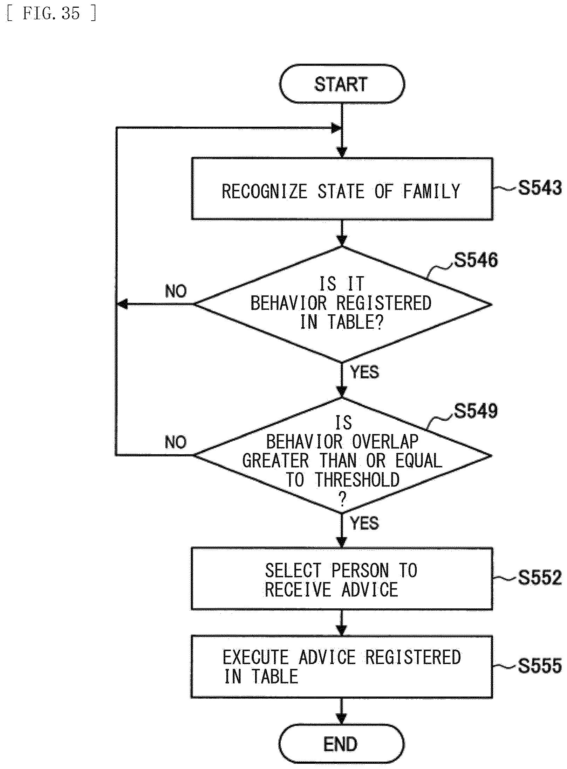

FIG. 35 is a flowchart for prompting adjustment (behavior modification of the life rhythm in accordance with overlapping of an event according to a modification example of the present embodiment. As illustrated in FIG. 35, first, the information processing apparatus 70 recognizes a state (behavior) of the family (step S543), and determines whether or not the state is a behavior (causing an event to occur) registered in the table as indicated in Table 11 (step S546). For example, the overlapping of the wake-up time, the TV-viewing time, or the like with a large number of persons is assumed.

Next, if the behavior overlap is greater than or equal to a threshold (step S549/Yes), the information processing apparatus 70 selects a person to receive advice (step S552). The person may be selected from those who take the overlap behavior or those who do not take the overlap behavior. The condition under which the person is selected may be registered in advance as indicated in Table 11 for each expected event.

Thereafter, the information processing apparatus 70 executes the advice registered in the table for the selected person (step S555).

In the example described above, it is assumed that a large number of cameras and microphones are installed in the house and the situation of the family is routinely grasped, as described above; however, it is also possible to grasp the situation of the family even if a large number of cameras and microphones are not installed in the house, on the basis of information from, for example, a camera, a microphone, a motion sensor, and the like of a smartphone, a smart band, and the like owned by the user.

For example, the camera or the microphone of the smartphone is able to sense an evening meal time period and that a TV is being viewed. In addition, it is possible to acquire location information such as which room the smartphone or the like is at by radio waves of a smartphone or the like (thus, it is possible to roughly predict what is being done when the location is known, such as a bathroom, a toilet, a living room, or the like).

Further, regarding whether a person is in the toilet, it is possible to determine that the person is in a small sealed space, i.e., a toilet (or a bathroom) by detecting a sound of water flushing through the microphone of the smartphone brought into the toilet, or by detecting that reverberation of a sound is large and intervals of echoes are short.

In addition, it is also possible to determine whether the user has taken a bath or not on the basis of a user's appearance captured by the camera of the smartphone (hair is wet, pajamas are worn, a dryer is being used, etc.).

### [5. Hardware Configuration]

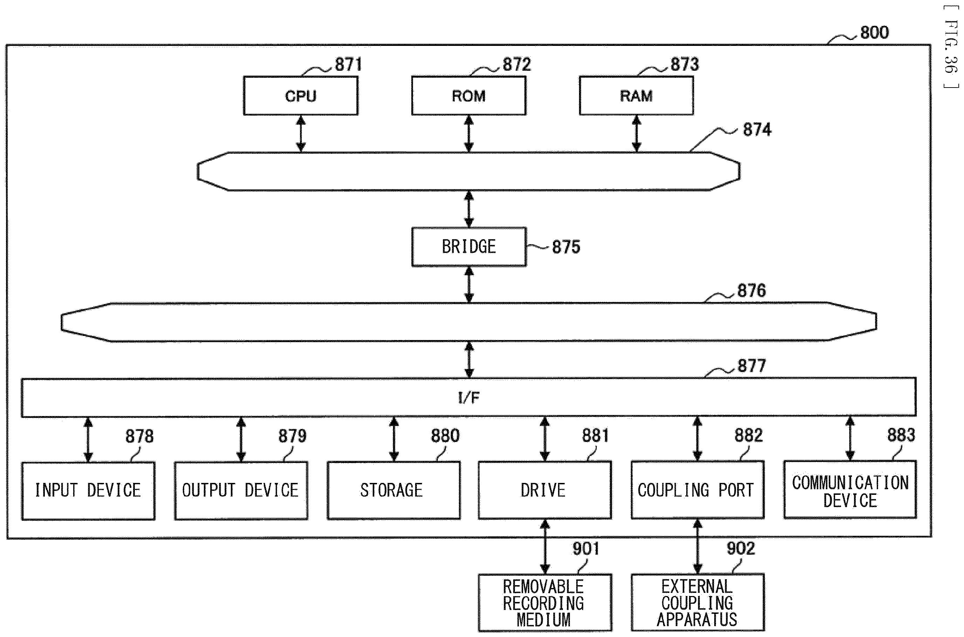

Finally, with reference to FIG. 36, a hardware configuration of an information processing apparatus according to the present embodiment will be described. FIG. 36 is a block diagram illustrating an example of a hardware configuration the information processing apparatus 20, the information processing apparatus 50, or the information processing apparatus 70 according to the present embodiment. It is to be noted that an information processing apparatus 800 illustrated in FIG. 36 may achieve the information processing apparatus 20, the information processing apparatus 50, or the information processing apparatus 70, for example. The information processing performed by the information processing apparatus 20, the information processing apparatus 50, or the information processing apparatus 70 according to the present embodiment is achieved by cooperation with software and hardware to be described below.

As illustrated in FIG. 36, the information processing apparatus 800 includes, for example, a CPU 871, a ROM 872, a RAM 873, a host bus 874, a bridge 875, an external bus 876, an interface 877, an input device 878, an output device 879, a storage 880, a drive 881, a coupling port 882, and a communication device 883. It is to be noted that the hardware configuration illustrated herein is merely an example, and a portion of the components may be omitted. In addition, a component other than the components illustrated herein may be further included.

### [CPU 871]

The CPU 871 functions as an arithmetic processing unit or a control unit, for example, and controls overall operations or a portion thereof of respective components on the basis of various programs recorded in the ROM 872, the RAM 873, the storage 880, or a removable recording medium 901.

Specifically, the CPU 871 achieves the operation process performed in the information processing apparatus 20, the information processing apparatus 50, or the information processing apparatus 70.

### [ROM 872 and RAM 873]

The ROM 872 is a means that stores programs to be read by the CPU 871 and data to be used for arithmetic operations. For example, a program to be read by the CPU 871, and various parameters that change appropriately when the program is executed, etc. are stored in the RAM 873 temporarily or permanently.

### [Host Bus 874, Bridge 875, External Bus 876, and Interface 877]

The CPU 871, the ROM 872, and the RAM 873 are coupled to one another, for example, via the host bus 874 that enables high-speed data transmission. Meanwhile, the host bus 874 is coupled to the external bus 876 having a relatively low data transmission speed, for example, via the bridge 875. In addition, the external bus 876 is coupled to various components via the interface 877.

### [Input Device 878]

For example, a mouse, a keyboard, a touch panel, a button, a switch, a lever, and the like are used as the input device 878. Further, a remote controller (hereinafter, a remote control) that is able to transmit a control signal utilizing infrared rays or other radio waves may also be used as the input device 878 in some cases. In addition, the input device 878 includes a sound input device such as a microphone.

### [Output Device 879]

The output device 879 is a device that is able to visually or auditorily notifying a user of acquired information, for example, a display device such as a CRT (Cathode Ray Tube), an LCD, or an organic EL, an audio output device such as a speaker or a headphone, a printer, a mobile phone, or a facsimile, etc. In addition, the output device 879 according to the present disclosures includes a variety of vibrating devices that are able to output tactile stimuli.

### [Storage 880]

The storage 880 is a device for storing various data. As the storage 880, for example, a magnetic storage device such as a hard disk drive (HDD), a semiconductor storage device, an optical storage device, a magneto-optical storage device, or the like is used.

### [Drive 881]

The drive 881 is, for example, a device that reads information recorded in the removable recording medium 901 or writes information into the removable recording medium 901, such as a magnetic disk, an optical disk, a magneto-optical disk, or a semiconductor memory.

### [Removable Recording Medium 901]

The removable recording medium 901 is, for example, a DVD medium, a Blu-ray (registered trademark) medium, a HD DVD medium, various semiconductor storage media, or the like. It is needless to say that the removable recording medium 901 may be, for example, an IC card mounted with a noncontact type IC chip, an electronic apparatus, or the like.

### [Coupling Port 882]

The coupling port 882 is, for example, a port for coupling of an external coupling apparatus 902, such as a USB (Universal Serial Bus) port, an IEEE 1394 port, an SCSI (Small Computer System Interface), an RS-232C port, or an optical audio terminal.

### [External Coupling Apparatus 902]

The external coupling apparatus 902 is, for example, a printer, a portable music player, a digital camera, a digital video camera, or an IC recorder. The external coupling apparatus 902 may also be, for example, the environment sensor 30, the user sensor 32, the output device 36, the sensor 60, the output device 62, the sensor 80, or the output device 82.

### [Communication Device 883]

The communication device 883 is a communication device for coupling to a network, and is, for example, a communication card for a wired or wireless LAN, Wi-Fi (registered trademark), Bluetooth (registered trademark), or a WUSB (Wireless USB), a router for optical communication, an ADSL (Asymmetric Digital Subscriber Line) router, or a modem for various communications.

### [6. Conclusion]

As described above, the information processing system according to an embodiment of the present disclosure is able to automatically generate a behavior rule of a community and to promote voluntary behavior modification.

A preferred embodiment(s) of the present disclosure has/have been described above in detail with reference to the accompanying drawings, but the technical scope of the present disclosure is not limited to such an embodiment(s). It is apparent that a person having ordinary skill in the art of the present disclosure can arrive at various alterations and modifications within the scope of the technical idea described in the appended claims, and it is understood that such alterations and modifications naturally fall within the technical scope of the present disclosure.

Further, it is also possible to create a computer program for causing hardware such as the CPU, the ROM, and the RAM, which are built in the information processing apparatus 20, the information processing apparatus 50, or the information processing apparatus 70, to exhibit functions of the information processing apparatus 20, the information processing apparatus 50, or the information processing apparatus 70. Further, there is also provided a storage medium having the computer program stored therein.

Furthermore, the effects described herein are merely illustrative and exemplary, and not limiting. That is, the technique according to the present disclosure can exert other effects that are apparent to those skilled in the art from the description herein, in addition to the above-described effects or in place of the above-described effects.

It is to be noted that the present disclosure may have the following configurations.
(1) An information processing apparatus including a controller that performs control to
   acquire sensor data obtained by sensing a member belonging to a specific community,
   automatically generate, on a basis of the acquired sensor data, a behavior rule in the specific community, and
   prompt, on a basis of the behavior rule, the member to perform behavior modification.
(2) The information processing apparatus according to (1), in which the controller
   estimates, on the basis of the acquired sensor data, an issue that the specific community has, and automatically generates the behavior rule that causes the issue to be solved.
(3) The information processing apparatus according to (1), in which the controller indirectly prompts the member to perform behavior modification to cause the member to perform behavior modification.
(4) The information processing apparatus according to (3), in which the controller
   sets a response variable as the behavior rule,
   generates a relationship graph indicating a relationship between factor variables having the response variable as a start point, and
   prompts the member to perform behavior modification on a factor variable to be intervened in which behavior modification is possible, out of factor variables associated with the response variable.
(5) The information processing apparatus according to (4), in which the controller encourages the member to cause a factor variable associated with the response variable to be approached to a desired value.
(6) The information processing apparatus according to (4), in which the controller
   generates a causal graph by estimating a factor variable that is estimated to be a cause of the response variable that is set as the behavior rule, and
   encourages the member to cause the factor variable that is estimated to be a cause of the response variable to be approached to a desired value.
(7) The information processing apparatus according to (3), in which the controller
   automatically generates, as a behavior rule, a sense of values to be a standard in the specific community, on the basis of the acquired sensor data, and
   indirectly prompts the member to perform behavior modification on a basis of the sense of values to be the standard.
(8) The information processing apparatus according to (7), in which the controller sets the sense of values to be the standard to an average of senses of values of a plurality of members belonging to the specific community.
(9) The information processing apparatus according to (7), in which the controller sets the sense of values to be the standard to a sense of values of a specific member out of the plurality of members belonging to the specific community.
(10) The information processing apparatus according to any one of (7) to (9), in which the controller indirectly prompts a specific member whose sense of values deviates from the sense of values to be the standard at a certain degree or more to perform behavior modification, by presenting the sense of values to be the standard to the specific member.
(11) The information processing apparatus according to any one of (2) to (6), in which the controller
   estimates, on the basis of the acquired sensor data, an issue that a member belonging to the specific community has, and
   automatically generates a behavior rule related to a life rhythm of the member belonging to the specific community to cause the issue to be solved.
(12) The information processing apparatus according to (11), in which the controller automatically generates a behavior rule that causes life rhythms of a plurality of members belonging to the specific community to be synchronized to cause the issue to be solved.
(13) The information processing apparatus according to (12), in which the controller indirectly prompts a specific member to perform behavior modification, the specific member having a life rhythm deviated from a life rhythm of another member belonging to the specific community for a certain time period or more.
(14) The information processing apparatus according to (11), in which the controller automatically generates a behavior rule that causes life rhythms of a plurality of members belonging to the specific community to be asynchronous to cause the issue to be solved.
(15) The information processing apparatus according to (14), in which, when it is detected that a certain number of members or more out of the plurality of members belonging to the specific community are synchronized with each other in a first life behavior, the controller indirectly prompts a specific member belonging to the specific community to perform behavior modification to cause a second life behavior to be performed, the second life behavior being predicted to come after the first life behavior.
(16) An information processing apparatus including a controller that performs control to encourage a member belonging to a specific community to perform behavior modification,
   depending on a behavior rule in the specific community, the behavior rule being automatically generated in advance on a basis of sensor data obtained by sensing the member belonging to the specific community,
   in accordance with the sensor data obtained by sensing the member belonging to the specific community.
(17) An information processing method performed by a processor, the method including:
   acquiring sensor data obtained by sensing a member belonging to a specific community;
   automatically generating, on a basis of the acquired sensor data, a behavior rule in the specific community; and
   prompting, on a basis of the behavior rule, the member to perform behavior modification.
(18) A recording medium having a program recorded therein, the program causing a computer to function as a controller that performs control to
   acquire sensor data obtained by sensing a member belonging to a specific community,
   automatically generate, on a basis of the acquired sensor data, a behavior rule in the specific community, and
   prompt, on a basis of the behavior rule, the member to perform behavior modification.

### Reference Signs List

- 2A to 2C: community
- 10, 10A to 10C, 10-1 to 10-3: master system
- 11: data analyzer
- 12: behavior rule generator
- 13: behavior modification instruction section
- 20: information processing apparatus (causality analysis server)
- 30: environment sensor
- 32: user sensor
- 34: service server
- 36: output device
- 50: information processing apparatus
- 70: information processing apparatus
- 80: sensor
- 82: output device
- 201: receiver
- 203: transmitter
- 210: image processor
- 212: voice processor
- 214: sensor/behavior data processor
- 220: factor variable DB
- 222: issue index DB
- 224: intervention device DB
- 226: intervention rule DB
- 228: intervention reservation DB
- 230: issue estimation section
- 232: causality analyzer
- 235: intervention section
- 500: controller
- 501: user management section
- 502: sense-of-values estimation section
- 503: sense-of-values comparison section
- 504: presentation section
- 510: communication section
- 520: storage
- 700: controller
- 701: person recognition section
- 702: action recognition section
- 703: rhythm derivation section
- 704: deviation detector
- 705: deviation-cause estimation section
- 706: response generator
- 710: communication section
- 720: storage

## Claims

1. An information processing apparatus comprising a controller that
acquires sensor data obtained by sensing a member belonging to a specific community,
automatically generates, on a basis of the acquired sensor data, a behavior rule in the specific community, and
performs control to prompt, on a basis of the behavior rule, the member to perform behavior modification.

2. The information processing apparatus according to claim 1, wherein the controller
estimates, on the basis of the acquired sensor data, an issue that the specific community has, and automatically generates the behavior rule that causes the issue to be solved.

3. The information processing apparatus according to claim 1, wherein the controller indirectly prompts the member to perform behavior modification to cause the member to perform behavior modification.

4. The information processing apparatus according to claim 3, wherein the controller
sets a response variable as the behavior rule,
generates a relationship graph indicating a relationship of factor variables having the response variable as a start point, and
prompts the member to perform behavior modification on a factor variable to be intervened in which behavior modification is possible, out of factor variables associated with the response variable.

5. The information processing apparatus according to claim 4, wherein the controller encourages the member to cause a factor variable associated with the response variable to be approached to a desired value.

6. The information processing apparatus according to claim 4, wherein the controller
generates a causal graph by estimating a factor variable that is estimated to be a cause of the response variable that is set as the behavior rule, and
encourages the member to cause the factor variable that is estimated to be a cause of the response variable to be approached to a desired value.

7. The information processing apparatus according to claim 3, wherein the controller
automatically generates, as a behavior rule, a sense of values to be a standard in the specific community, on the basis of the acquired sensor data, and
indirectly prompts the member to perform behavior modification on a basis of the sense of values to be the standard.

8. The information processing apparatus according to claim 7, wherein the controller sets the sense of values to be the standard to an average of senses of values of members belonging to the specific community.

9. The information processing apparatus according to claim 7, wherein the controller sets the sense of values to be the standard to a sense of values of a specific member out of the members belonging to the specific community.

10. The information processing apparatus according to claim 7, wherein the controller indirectly prompts a specific member whose sense of values deviates from the sense of values to be the standard at a certain degree or more to perform behavior modification, by presenting the sense of values to be the standard to the specific member.

11. The information processing apparatus according to claim 2, wherein the controller
estimates, on the basis of the acquired sensor data, an issue that a member belonging to the specific community has, and
automatically generates a behavior rule related to a life rhythm of the member belonging to the specific community to cause the issue to be solved.

12. The information processing apparatus according to claim 11, wherein the controller automatically generates a behavior rule that causes life rhythms of members belonging to the specific community to be synchronized to cause the issue to be solved.

13. The information processing apparatus according to claim 12, wherein the controller indirectly prompts a specific member to perform behavior modification, the specific member having a life rhythm deviated from a life rhythm of another member belonging to the specific community for a certain time period or more.

14. The information processing apparatus according to claim 11, wherein the controller automatically generates a behavior rule that causes life rhythms of members belonging to the specific community to be asynchronous to cause the issue to be solved.

15. The information processing apparatus according to claim 14, wherein, when a certain number of members out of the members belonging to the specific community are synchronized with each other in a first life behavior, the controller indirectly prompts a specific member belonging to the specific community to perform behavior modification to cause a second life behavior to be performed, the second life behavior being predicted to come after the first life behavior.

16. An information processing apparatus comprising a controller that encourages a member belonging to a specific community to perform behavior modification,
depending on a behavior rule in the specific community, the behavior rule being automatically generated in advance on a basis of sensor data obtained by sensing the member belonging to the specific community,
in accordance with the sensor data obtained by sensing the member belonging to the specific community.

17. An information processing method performed by a processor, the method comprising:
acquiring sensor data obtained by sensing a member belonging to a specific community;
automatically generating, on a basis of the acquired sensor data, a behavior rule in the specific community; and
performing control to prompt, on a basis of the behavior rule, the member to perform behavior modification.

18. A recording medium having a program recorded therein, the program causing a computer to function as a controller that
acquires sensor data obtained by sensing a member belonging to a specific community, automatically generates, on a basis of the acquired sensor data, a behavior rule in the specific community, and
performs control to prompt, on a basis of the behavior rule, the member to perform behavior modification.
